# FASCICULE DE BREVET EUROPEEN

(11) **EP 0 501 892 B1**
(45) Date de publication et mention de la délivrance du brevet: **24.07.1996**
(21) Numéro de dépôt: 92400523.4
(22) Date de dépôt: 28.02.1992
(51) Int. Cl.: C07D 235/02, C07D 233/70, C07D 239/22, C07D 403/10, C07D 413/10, C07D 417/10, C07D 239/70

(54) **Dérivés hétérocycliques diazotés N-substitués par un groupement biphénylméthyle, leur préparation, les compositions pharmaceutiques en contenant**
Diazotierte heterocyclische Derivate aus Stickstoff, substituiert durch eine Biphenylgruppe, ihre Herstellung, und diese enthaltende pharmazeutische Zubereitungen
Diazotized heterocyclic derivatives N-substituted by a biphenylmethyl group, their preparation and pharmaceutical compositions containing them

(30) Priorité: 01.03.1991 FR 9102501
(43) Date de publication de la demande: 02.09.1992
(73) Titulaire: SANOFI, F-75008 Paris (FR)
(72) Inventeur: Bernhart, Claude, F-34980 Saint Gely du Fesc (FR); Ferrari, Bernard, F-34980 Saint Gely du Fesc (FR); Perreaut, Pierre, F - 34980 Saint Gély du Fesc (FR)
(74) Mandataire: Gillard, Marie-Louise

(56) Documents cités:
- EP-A- 0 380 959
- EP-A- 0 401 030
- EP-A- 0 407 342
- EP-A- 0 411 766
- EP-A- 0 454 511
- EP-A- 0 459 136
- US-A- 4 675 403

## Description

La présente invention concerne des dérivés hétérocycliques diazotés N-substitués, leur préparation et les compositions pharmaceutiques en contenant.

Les composés selon l'invention antagonisent l'action de l'angiotensine II qui est une hormone peptidique de formule :
H-Asp-Arg-Val-Tyr-Ile-His-Pro-Phe-OH

L'angiotensine II est un puissant vasopresseur qui est le produit biologiquement actif du système rénine-angiotensine : la rénine agit sur l'angiotensinogène du plasma pour produire l'angiotensine I, celle-ci est convertie en angiotensine II par action de l'enzyme de conversion de l'angiotensine I.

Les composés de la présente invention sont des composés non peptidiques, antagonistes de l'angiotensine II. En inhibant l'action de l'angiotensine II sur ses récepteurs, les composés selon l'invention empêchent notamment l'augmentation de la pression sanguine produite par l'interaction hormone-récepteur, ils ont également d'autres actions physiologiques au niveau du système nerveux central.

Ainsi les composés selon l'invention sont utiles dans le traitement d'affections cardiovasculaires comme l'hypertension, la défaillance cardiaque ainsi que dans le traitement d'affections du système nerveux central et dans le traitement du glaucome et de la rétinopathie diabétique.

La présente invention a pour objet des composés de formule : 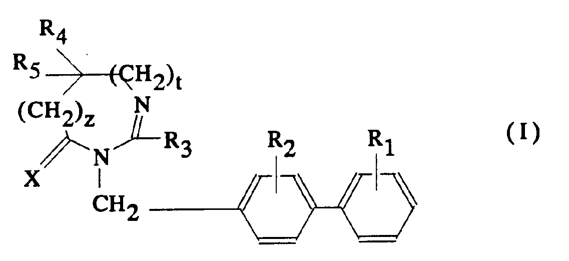 dans laquelle :
R₁ est en position ortho et représente un hétérocycle azoté choisi parmi :

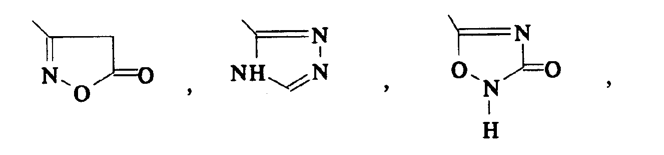

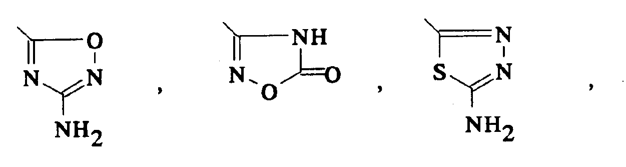

- R₂ est l'hydrogène ;
R₃ représente un alkyle en C₁-C₆ ;
- R₄ et R₅ liés ensemble avec le carbone auquel ils sont attachés, représentent un groupe de formule (CH₂)ₙ ;
- n est égal à 4 ou 5 ;
- z et t sont nuls ou z représente 1 et t est nul ;
- X représente un atome d'oxygène ;

et leurs sels.

Les sels éventuels des composés de formule (I) selon la présente invention comprennent ceux avec des acides minéraux ou organiques qui permettent une séparation ou une cristallisation convenable des composés de formule (l), tels que l'acide trifluoroacétique, l'acide picrique, l'acide oxalique ou un acide optiquement actif, par exemple un acide mandélique ou un acide camphosulfonique, et ceux qui forment des sels pharmaceutiquement acceptables tels que le chlorhydrate, le bromhydrate, le sulfate, l'hydrogénosulfate, le dihydrogénophosphate, le méthanesulfonate, le méthyl- sulfate, le maléate, le fumarate, le 2-naphtalènesulfonate.

Les sels des composés de formule (l) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente description et dans les revendications qui vont suivre, par atome d'halogène on entend un atome de brome, de chlore ou de fluor ; par groupe carboxy estérifié on entend un ester labile dans des conditions appropriées, comme par exemple un ester méthylique, éthylique, benzylique ou tertiobutylique.

Les hétérocycles azotés qui sont décrits dans la définition des substituants R₁ et R₂ peuvent également exister sous plusieurs formes tautomères, chacune des formes tautomères fait partie de l'invention.

Les abréviations suivantes sont utilisées dans la description et dans les exemples :

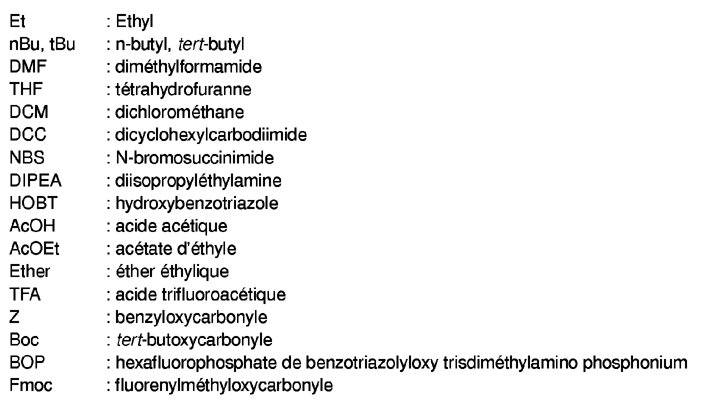

La présente invention a également pour objet le procédé de préparation des composés (1). Ledit procédé est caractérisé en ce que :
a) on fait réagir un dérivé hétérocyclique de formule : 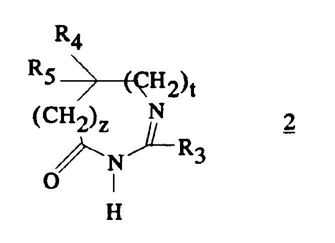 dans laquelle, z, t, R₃, R₄ et R₅ ont les significations indiquées ci-dessus pour (I), sur un dérivé du (biphényl-4-yl) méthyle de formule : 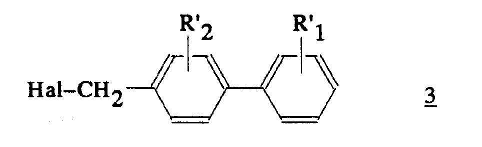 dans laquelle Hal représente un atome d'halogène et R'₁ et R'₂ représentent respectivement soit R₁ et R₂, soit un précurseur de R₁ et/ou R₂
b) le composé ainsi obtenu de formule : 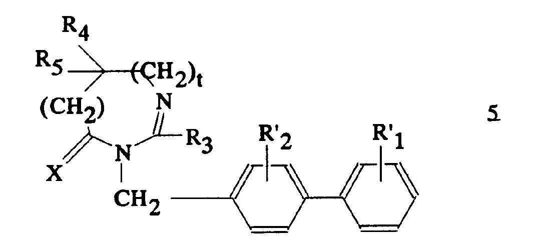 dans laquelle X représente un atome d'oxygène ou un atome de soufre, est traité pour préparer le composé (I) par transformation des groupes R'₁ et/ou R'₂ en respectivement R₁ et/ou R₂.

Le composé 2 est connu ou préparé par des méthodes connues. Par exemple, on peut utiliser la méthode décrite par Jacquier et al. (Bull. Soc. Chim France, 1971, (3), 1040-1051) et faire agir un imidate d'alkyle 6 sur un acide aminé ou son ester 5', selon le schéma réactionnel suivant : 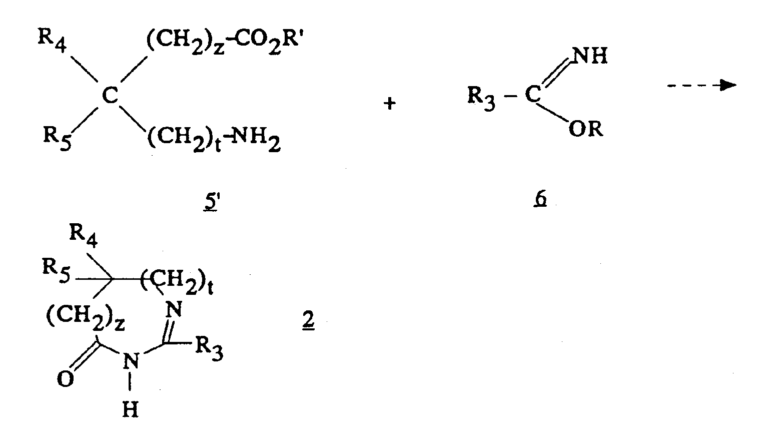 dans lequel R représente un alkyle en C₁-C₄ et R' représente l'hydrogène ou un alkyle en C₁-C₄, z, t, R₃, R₄ et R₅ sont tels que définis précédemment.

Cette réaction est effectuée en milieu acide, par chauffage dans un solvant inerte tel que le xylène ou le toluène.

Les composés 5' sont des composés connus ou préparés par des méthodes connues. Lorsque R₄ et R₅ sont différents, les composés 5' peuvent être obtenus optiquement purs en utilisant des méthodes de synthèse asymétrique ou des méthodes de résolution du mélange racémique telles que décrites dans Synthesis of Optically Active, a-ami- noacids, R.M. Williams, Pergamon Press. 1989.

Le dérivé du (biphényl-4-yl) méthyle (3) est préparé selon un procédé adapté de celui décrit dans la demande de brevet européen 324 377.

Ainsi la préparation d'un composé 3 dans lequel R'₁ et/ou R'₂ représente un groupe carboxy ou carboxyester est décrite dans la demande de brevet citée ci-dessus.

On utilise les méthodes connues de la littérature pour préparer des composés 3 dans lesquels R₁ et/ou R₂ ont l'une des valeurs décrites ci-dessus pour (l). L'obtention du groupement R₁ et/ou R₂ souhaité peut également être effectuée à la dernière étape du procédé décrit ci-dessus à partir du composé 5 dans lequel R'₁ et/ou R'₂ représente un groupe carboxy ou un dérivé d'un groupe carboxy. Un tel composé 5 est préparé à partir d'un composé 3 dans lequel R'₁ et/ou R'₂ représente un groupe permettant de préparer R₁ et/ou R₂.

On peut citer les références de la littérature ci-dessous pour la préparation des substituants R₁ et/ou R₂ des composés 3 ou 5:
- oxo-5 oxadiazol-1,2,4-yle-3 :
   . M.A. Perez et al., Synthesis, 1983, 483.
   . A.R. Katritzky et al., Tetrahedron, 1965, 21, 1681-1692.
   . K.R. Rao et al., Heterocycles, 1988, 27 (3), 683-685.
   . G. Babu et al., J. Org. Chem., 1976, 41 (20), 3233-3237.

Ce substituant est obtenu à partir du groupe C(OC₂H₅) = NC0₂CH₃ ; le substituant de départ est un groupe carboxyle transformé en groupe carboxamido, puis en carboximidate d'éthyle [R.J. Bergeron et al., J. Org; Chem., 1985, 50 (15) 2781].
- oxo-3 oxadiazol- 1,2,4-yle-5 :
   . A.R. Katritzky et al.,Tetrahedron, 1965, 21, 1681-1692.
   . Brevets allemands DE 2 312 500 et DE 2 212 797.
   O. Tsuge et al., J. Org. Chem., 1980, 45, 5130.

Ce substituant est obtenu à partir d'un carboxyle, transformé en groupe carboxamido, puis en groupe isocyanatocarbo- nyle.
- dihydro-4,5 oxo-5 isoxazolyle-3 :
   . F De Sarlo et al., Tetrahedron, 1966, 22, 2989-2994.

Ce substituant est obtenu à partir du groupe COCH₂CO₂C₂H₅, lequel est préparé à partir d'un carboxyle [W. Wierenga et al., J. Org. Chem., 1979, 44, 310-311].
- triazol-1,2,4-yle-3 :
   . H.Y. Yale et al., J. Med. Chem., 1966, 9, 42.

Ce substituant est obtenu à partir du carbonylthiosemicarbazide lequel est préparé à partir d'un groupe carboxyle [J. Sandstrom dans Advances in Heterocyclic Chemistry (A.R. Katritzky et A.J. Boulton ed.) Academic Press, 1968, 9, 175].
- amino-2 thiadiazol-1,3,4-yle-5 : ce substituant est également obtenu à partir du carbonylthiosemicarbazide ci-dessus.
- amino-3 oxadiazol-1,2,4,-yle-5 :
   J. Saunders et al., J. Chem. Soc. Chem., 1988, 1618.

Ce substituant est obtenu à partir du (O-guanidino) carboxylate, préparé par action d'hydroxy-2 guanidine sur un acide carboxylique en présence de BOP et de DIPEA.

Dans le procédé selon l'invention, l'étape a) est réalisée dans un solvant inerte tel que le DMF, le DMSO ou le THF, en milieu basique, par exemple en présence de potasse, de carbonate de potassium, d'un alcoolate métallique, d'un hydrure métallique ou de triéthylamine.

L'affinité des produits selon l'invention pour les récepteurs de l'angiotensine Il a été étudiée sur un test de liaison de l'angiotensine Il marquée à l'iode 125 à des récepteurs membranaires de foie de rats. La méthode utilisée est celle décrite par S. Keppens et al. dans Biochem. J., 1982, 208, 809-817.

On mesure la Ciₛₒ : concentration qui donne 50% de déplacement de l'angiotensine Il marquée, liée spécifiquement au récepteur. La C1₅₀ des composés selon l'invention est inférieure à 10-⁶M.

De plus l'effet antagoniste de l'angiotensine Il des produits selon l'invention a été constaté sur différentes espèces animales dans lesquelles le système rénineangiotensine a été préalablement activé (C. Lacour et al., J. Hypertension, 1989, 7 (suppl.2), S33-S35).

Les composés selon l'invention sont actifs après administration par différentes voies, notamment par voie orale.

Aucun signe de toxicité n'est observé pour ces composés aux doses pharmacologiquement actives.

Ainsi les composés selon l'invention peuvent être utilisés dans le traitement de différentes affections cardiovasculaires, notamment l'hypertension, la défaillance cardiaque, les insuffisances veineuses, ainsi que dans le traitement du glaucome, des rétinopathies diabétiques et de différentes affections du système nerveux central, l'anxiété, la dépression, les déficits mnésiques ou la maladie d'Alzheimer par exemple.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaité.

Dans les compositions pharmaceutiques de la présente invention pour l'administration orale, sublinguale, sous- cutanée, intramusculaire, intraveineuse, topique, intratrachéale, intranasale, transdermique ou rectale, les principes actifs de formule 1 ci-dessus, ou leurs sels éventuels, peuvent être administrés sous forme unitaire d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres,les granules et les solutions ou suspensions orales, les formes d'administration sublinguale, buccale, intratrachéale, intranasale, les formes d'administration sous- cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule 1 ci-dessus ou d'un de leurs sels pharmaceutiquement acceptables, d'autres principes actifs tels que, par exemple, des tranquillisants ou d'autres médicaments qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention et le ou les autres peuvent être un composé béta-bloquant, un antagoniste calcique, un diurétique, un antiinflammatoire non stéroidien ou un tranquillisant.

Les exemples suivants illustrent l'invention sans toutefois la limiter. Dans ces exemples, on utilise les abréviations suivantes :
TA signifie température ambiante, KHSO₄-K₂SO₄ signifie une solution aqueuse contenant 16,6 g de bisulfate de potassium et 33,3 g de sulfate de potassium pour 1 litre. D'une façon générale, les solutions salines employées sont des solutions aqueuses.

Les points de fusion (Fc) sont donnés en degrés Celsius ; sauf indication contraire, ils sont mesurés sans recristallisation du produit.

La pureté des produits est vérifiée en chromatographie sur couche mince (CCM) ou par CLHP (chromatographie liquide à haute performance). Les produits sont caractérisés par leur spectre RMN enregistré à 200 MHz dans le DMSO deutérié, la référence interne étant le tétraméthylsilane, sauf indication contraire.

Pour l'interprétation des spectres de RMN, on emploie :

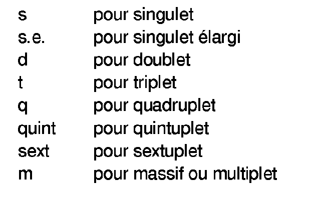

### Par ailleurs, im signifie imidazole.

De façon classique, pour les composés exemplifiés ci-après, les atomes d'hydrogène sont numérotés sur le biphényle comme représenté dans la formule suivante :

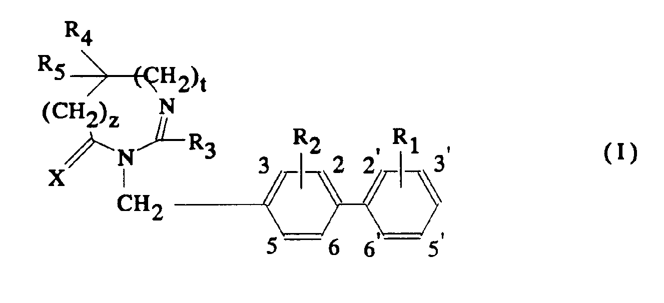

Dans les exemples ci-dessous, z et t sont nuls.

### EXEMPLES 1 ET 2

n-butyl-2 [((oxo-5 oxadiazol-1,2,4-yl-3)2' biphényl-4-yl) méthyl]-1 spirocyclopentane-4 imidazoline-2 one-5. (1 : R₂ = H, 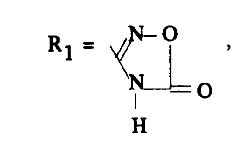
R₃ = n-C₄H₉, CR₄R₅ = cyclopentane, X = O). et
[(n-butyl-2 oxo-5 spirocyclopentane-4) imidazolin-2-yl-1] méthyl-4' biphényl-2 (N-méthoxycarbonyl) carboximidate d'éthyle.
(1 : R₂ = H, 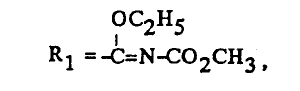
R₃ = n-C₄H₉, CR₄R₅ = cyclopentane, X = O).
A) n-butyl-2 spirocyclopentane-4 imidazoline-2 one-5.

L'ester éthylique de l'acide amino-1 cyclopentane carboxylique est préparé selon Adkins et Billica (J. Amer. Chem. Soc., 1948, 70, 3121).

Le valérimidate d'éthyle est préparé selon Mac Elvain (J. Amer Chem. Soc., 1942, 64, 1825-1827) puis libéré de son chlorhydrate par action du carbonate de potassium et extraction par le DCM.

L'ester éthylique de l'acide amino-1 cyclopentane carboxylique (1,57 g) et le valérimidate d'éthyle (1,56 g) sont dissous dans 12 ml de xylène contenant 6 gouttes d'acide acétique. Après 6 heures et demie de chauffage à reflux, le milieu réactionnel est concentré sous vide puis le résidu est chromatographié sur gel de silice en éluant par un mélange chloroforme/méthanol/acide acétique (94/4/2 ; v/v/v). Le fraction contenant le produit attendu est évaporée plusieurs fois en présence de xylène puis de benzène pour éliminer l'acide acétique. On obtient 1,91 g de produit sous forme d'une huile épaisse.
- IR(CHC1₃) :
   1720 cm⁻¹: C = 0
   1635 cm⁻¹ : C = N

Remarque : le fait de ne pas voir de bande entre 1500 et 1600 cm⁻¹ indique que, dans la solution de chloroforme, le produit est une imidazolinone-5.
- Spectre RMN :
   0,92 ppm : t : 3 H : CH₃ (nBu)
   1,35 ppm : sext : 2 H : CH₃-CH₂-
   1,50-1,93 ppm : m : 10 H : CH₃-CH₂-CH₂ et cyclopentane
   2,33 ppm : t : 2 H : CH₃-CH₂-CH₂-CH₂-
   10,7 ppm : m : NH
- Spectre de masse : MH⁺ = 195.

### B) méthyl-4 biphényl-2' (N-méthoxycarbonyl) carboximidate d'éthyle

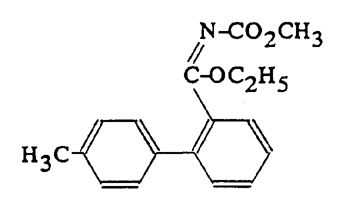

La préparation de ce composé est réalisée selon R.J. Bergeron et al., J. Org. Chem., 1985, 50 (15), 2781 (préparation de l'imidate) puis M.A. Perez, Synthesis, 1983, 483.

Le méthyl-4'biphényl-2 carboxamide est préparé selon la demande de brevet européen 253 310.

3,09 g de ce composé sont mis en solution dans 100 ml de chlorure de méthylène et traités par 4,43 g d'hexafluo- rophosphate de triéthyloxonium à 90% dans l'éther éthylique, pendant 24 heures à température ambiante. On ajoute 50 ml d'un mélange eau-glace et on extrait la phase organique puis on la lave par 50 ml de carbonate de sodium 1 M puis 20 ml d'eau. Après séchage sur sulfate de sodium, la solution est filtrée et concentrée. On obtient une huile qui cristallise à froid. On reprend par 50 ml d'hexane et on traite par 1,36 ml de chloroformiate de méthyle et 2,06 ml de triméthyl-2,4,6 pyridine à reflux pendant 24 heures. Le solide obtenu est essoré et rincé par de l'hexane. Le filtrat est concentré et purifié par chromatographie sur silice en éluant par un mélange hexane-acétate d'éthyle (6/4, v/v). L'huile obtenue cristallise lentement au froid. On obtient 3,53 g du composé attendu.

Fc = 54-57°C.
- Spectre RMN :
   0,9 ppm : t : 3 H : CH₂-CH₃
   2,3 ppm : s : 3 H : C₆H₄-CH₃
   3,5 ppm : s : 3 H : CO₂-CH₃
   3,9 ppm : q : 2H : -CH₂-CH₃

### C) bromométhyl-4 biphényl-2'(N-méthoxycarbonyl) carboximidate d'éthyle

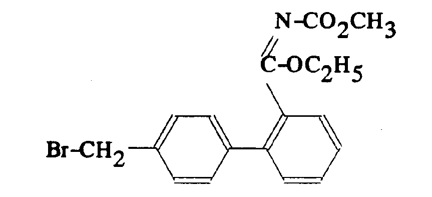

1,008 g du composé obtenu à l'étape précédente sont mis en solution dans 40 ml de tétrachlorocarbone puis traités par 610 mg de N-bromosuccinimide et 15 mg d'azobisisobutyronitrile pendant 3 heures au reflux. Après refroidissement, le milieu réactionnel est filtré et le filtrat est concentré. Le composé attendu est obtenu sous forme d'une huile dont la pureté est 70%.
- Spectre RMN :
   0,9 ppm : t : 3 H : CH₂-CH₃
   3,55 ppm : s : 3 H : CO₂CH₃
   3,9 ppm : q : 2 H : -CH₂-CH₃
   4,8 ppm : s : 2 H : -CH₂-Br

### D) [(n-butyl-2 oxo-5 spirocyclopentane-4) imidazolin-2-yl-1] méthyl-4' biphényl-2 (N-méthoxycarbonyl) carboximidate d'éthyle.

781 mg d'imidazolinone préparée à l'étape A sont utilisés sous forme de chlorhydrate, en solution dans 20 ml de diméthylformamide et traités sous azote par 223 mg d'hydrure de sodium à 80% dans l'huile. Après 15 minutes sous agitation à température ambiante, on ajoute par fractions le dérivé bromé obtenu à l'étape C (1,3 g) en solution dans 10 ml de diméthylformamide et on laisse 2 heures sous agitation. Après concentration du milieu réactionnel, l'huile obtenue est reprise dans 100 ml d'acétate d'éthyle et lavée 2 fois par 30 ml d'une solution de KHSO₄-K₂SO₄, 30 ml d'une solution saturée de chlorure de sodium, 30 ml d'une solution saturée d'hydrogénocarbonate de sodium et à nouveau 30 ml de solution saturée de chlorure de sodium. Après séchage sur sulfate de sodium, la solution est concentrée et l'huile obtenue est purifiée par chromatographie sur silice en éluant par un mélange heptane-acétate d'éthyle (8/2 puis 7/3, v/v). Les fractions pures sont concentrées et l'on obtient le produit attendu sous forme d'une huile jaune : m = 1,1 g.
- Spectre RMN :
   0,9 ppm : quint : 6 H : CH₂-CH₃(OEt), CH₃ (nBu)
   1,3 ppm : sext : 2 H : CH₂-CH₃ (nBu)
   1,55 ppm : quint : 2 H :CH₂-CH₂-CH₃ (nBu)
   1,75-2 ppm : m :8 H : cyclopentane
   2,40 ppm : t : 2 H : CH₂-CH₂-CH₂-CH₃
   3,55 ppm : s : 3 H : CO₂-CH₃
   3,95 ppm : q : 2 H : CH₂-CH₃(OEt)
   4,8 ppm : s : 2 H : CH₂-C₆H₄-
   7,2-7,7 ppm : m : 8 H : aromatiques

### E) Cette étape est réalisée selon M.A. Perez, Synthesis, 1983, 483.

n-butyl-2 [((oxo-5 oxadiazol-1,2,4-yl-3)-2' biphényl-4-yl) méthyl]-1 spirocyclo-pentane-4 imidazoline-2 one-5.

On traite 76 mg de chlorhydrate d'hydroxylamine dans 5 ml de méthanol à température ambiante pendant 15 minutes par 62 mg de méthylate de sodium à 95%. On ajoute 490 mg du composé obtenu à l'étape D dans 5 ml de méthanol; Après une nuit de chauffage au reflux, le milieu réactionnel est concentré et repris par du dichlorométhane. Un insoluble est éliminé et le filtrat est concentré puis chromatographié sur silice en éluant par un mélange heptane-acétate d'éthyle (4/6 puis 3/7, v/v). Les fractions pures sont réunies et concentrées pour donner un solide blanc qui est repris par de l'hexane, essoré et séché.
m = 340 mg
Fc ₌ 157-160°C

- Spectre RMN :
   0,9 ppm t : 3 H : CH₃ (nBu)
   1,3 ppm : sext : 2 H : CH₂-CH₃ (nBu)
   1,5 ppm : quint : 2 H :CH₂-CH₂-CH₃ (nBu)
   1,75-1,95 ppm : m : 8 H : cyclopentane
   2,35 ppm : t : 2 H : CH₂-CH₂-CH₂-CH₃ (nBu)
   4,75 ppm : s : 2 H : CH₂-C₆H₄-
   7,2-7,8 ppm : m : 8 H : aromatiques
   12,4 ppm : s : 1 H : NH

### EXEMPLES 3 ET 4

### [((dihydro-4,5 oxo-5 isoxazol-3-yl)-2' biphényl-4-yl) méthyl]-1 n-propyl-2 spirocyclohexane-4 imidazoline-2 one-5. (I: R₂ = H,

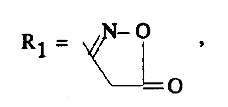

R₃ = n-C₃ H₇, CR₄R₅ = cyclohexane , X = O).

et [((n-propyl-2 oxo-5 spirocyclohexane-4) imidazolin-2-yl-1) méthyl-4' biphényl-2-yl]-3 oxo-3 propionate d'éthyle. (I : R₂ = H, R₁ = -COCH₂CO₂C₂H₅, R₃ = n-C₃H₇, CR₄R₅ = cyclohexane, X = O).

### A) Chlorhydrate de butyrimidate d'éthyle

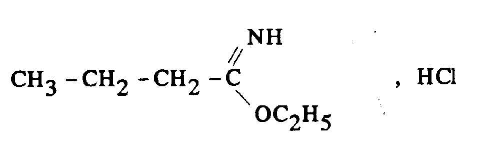

Ce composé est préparé selon Mc Elvain (J. Amer. Chem. Soc., 1942, 64, 1825-1827).

A une solution de 10,6 g de gaz chlorhydrique dans 20 ml d'éthanol anhydre, on ajoute à 0°C 23 ml de butyronitrile puis après abandon du milieu réactionnel 4 jours à 0°C, on le verse, sous agitation, sur 200 ml d'éther anhydre à 0°C ; le précipité formé est filtré, lavé à l'éther puis séché sous vide. On obtient 25,8 g du produit attendu.

### B) Butyrimidate d'éthyle.

16 g d'imidate obtenu à l'étape A sont dissous dans 100 ml de dichlorométhane et 50 ml d'eau et on ajoute 15 g de carbonate de potassium. Après décantation, le dichlorométhane est séché sur carbonate de potassium purs évaporé à sec sans chauffer.

### C) Ester éthylique de l'acide amino-1 cyclohexane carboxylique.

L'acide amino-1 cyclohexane carboxylique est commercial. 15 g de cet aminoacide sont ajoutés à 0°C à une solution de 23 g de gaz chlorhydrique dans 150 ml d'éthanol anhydre. On chauffe à reflux pendant 5 heures, puis on concentre à sec le milieu réactionnel et on le reprend par de l'éther. Le solide blanc obtenu est filtré, lavé à l'éther puis dissous dans un mélange de 300 ml d'éther et 100 ml d'eau. On amène à pH 9 par addition d'une solution de carbonate de potassium. Le phase organique est décantée, lavée par une solution saturée de chlorure de sodium, séchée sur sulfate de sodium puis évaporée à sec. On obtient 14 g du produit attendu sous forme d'une huile.

### D) n-propyl-2 spirocyclohexane-4 imidazoline-2 one-5.

14 g du produit obtenu à l'étape C sont dissous dans 200 ml de xylène contenant 0,6 ml d'acide acétique. On ajoute la moitié de l'imidate obtenu à l'étape B et on chauffe à reflux. Après 1 heure et demie, on ajoute la moitié de l'imidate restant puis le dernier quart après 4 heures. Après 7 heures de reflux au total, le milieu est évaporé à sec. Le solide obtenu est repris dans de l'hexane, filtré, lavé à l'éther puis séché.

On obtient 10,3 g de l'imidazolinone attendue.

Fc = 124-125°C.
- IR (CHCI₃)
   1715cm⁻¹ :C=0
   1635 cm⁻¹ : C = N

note : Le composé présent dans la solution est bien une imidazolinone-5, d'après les valeurs des bandes IR.

### E) n-propyl-2 spirocyclohexane-4 [(tert butoxycarbonyl-2' biphényl-4-yl) méthyl]-1 imidazoline-2 one-5.

A 0,24 g d'hydrure de sodium à 80% dans l'huile, en suspension dans 10 ml de diméthylformamide, on ajoute 970 mg d'imidazolinone obtenue à l'étape D. Après 20 minutes d'agitation sous azote, on ajoute en 5 minutes 1,91 g de bromométhyl-4 tert-butoxycarbonyl-2' biphényle, préparé selon la demande de brevet européen 324 377. Après 1 heure d'agitation, le milieu est concentré sous vide de moitié et repris par 100 ml d'acétate d'éthyle puis par 20 ml d'eau. La phase organique est décantée, lavée par une solution saturée de chlorure de sodium, séchée sur sulfate de sodium puis concentrée sous vide. Le résidu est chromatographié sur silice en éluant par un mélange acétate d'éthyle-toluène. On obtient 2,10 g du produit attendu sous forme de cire.
- IR (CHCI₃)
   1705-1715 cm⁻¹ : C = 0
   1635cm⁻¹ :C=N

L'analyse du spectre RMN confirme la structure.

### F) [(carboxy-2' biphényl-4-yl) méthyl]-1 n-propyl-2 spirocyclohexane-4 imidazoline-2 one-5.

1,25 g de l'ester tertiobutylique obtenu à l'étape E sont agités 45 minutes dans un mélange de 11 ml de dichlorométhane et 15 ml d'acide trifluoroacétique. Après concentration sous vide, le résidu est repris dans de l'éther. Le solide formé est filtré, lavé à l'éther puis séché. On obtient 1,04 g de solide blanc.

Fc = 170-172°C
- Spectre RMN :
   7,10-7,80 ppm : m : 8 H : aromatiques
   4,90 ppm : s : 2 H : CH₂-C₆H₄-
   2,45 ppm : t : 2 H : CH₃-CH₂-CH₂-
   1,40-1,80 ppm : m : 12 H : spirocyclohexane + CH₃-CH₂-CH₂-
   0,90 ppm : t : 3 H : CH₃-CH₂-CH₂-

1,60 g du trifluoroacétate obtenu précédemment sont dissous dans 150 ml d'acétate d'éthyle plus 20 ml d'eau. On ajoute de la soude 1 N pour obtenir pH 5,0. La phase organique est décantée, lavée par une solution saturée de chlorure de sodium, séchée sur sulfate de sodium puis évaporée à sec. Le résidu solide est repris dans de l'éther éthylique, filtré et séché.
m=1,14g
Fc = 208-210°C.

### G) [((n-propyl-2 oxo-5 spirocyclohexane-4) imidazolin-2-yl-1) méthyl-4' biphényl-2-yl]-3 oxo-3 propionate d'éthyle.

(I: R₂ = H, R₁ = -COCH₂CO₂C₂H₅, R₃ = n-C₃H₇, CR₄R₅ = cyclohexane, X = O).

Ce composé est préparé à partir de l'acide obtenu à l'étape F selon la méthode décrite par W Wierenga et H.I. Skulnick dans J. Org. Chem., 1979, 44, 310-311.

A 500 mg de l'acide obtenu à l'étape précédente, en suspension dans 7 ml de dichlorométhane, on ajoute 0,9 ml de chlorure de thionyle. Après 1 heure 20 minutes, la solution obtenue est concentrée sous vide puis évaporée 2 fois avec du benzène. On obtient le chlorure d'acide sous forme d'une mousse blanche. A 402 mg de monomalonate d'éthyle dans 6 ml de THF, on ajoute à -70°C en 5 minutes, 3,75 ml de n-butyllithium 1,6 M dans l'hexane, on laisse remonter la température à -5°C, puis on refroidit de nouveau à -65°C et on ajoute en 10 minutes le chlorure d'acide dissous dans 5 ml de THF On laisse la température remonter en 1 heure à 0°C et on agite pendant 20 heures à 0°C. Le milieu réactionnel est versé sur 100 ml d'acétate d'éthyle contenant 9 ml d'acide chlorhydrique 1 N et on agite 10 minutes puis on amène à pH 8,0 par addition de soude 1 N. La phase organique est décantée, lavée par une solution saturée de chlorure de sodium, séchée sur sulfate de sodium puis concentrée sous vide. Le résidu est chromatographié sur silice, en éluant par un mélange acétate d'éthyle-toluène. On obtient 300 mg du produit attendu sous forme d'une huile.

IR (CHCI₃)

1710-1730 cm-¹ : 3 carbonyles sous forme d'une bande large.

1630 cm-¹ : C = N de l'imidazolinone.
- Spectre RMN :
   7,10-7,70 ppm : m : 8 H : aromatiques
   4,65 ppm : s : 2 H : CH₂-C₆H₄-
   3,95 ppm q : 2 H : CH₂ de l'ester éthylique
   3,60 ppm s : 2 H : CH₂ du β-cétoester
   2,25 ppm : t : 2 H : CH₃-CH₂-CH₂
   1,20-1,70 ppm : m : 12 H : 10 H du cyclohexane + 2H (CH₃-CH₂-CH₂-)
   1,05 ppm : t : 3 H : CH₃ de l'ester éthylique
   0,80 ppm : t : 3 H : CH₃-CH₂-CH₂

Note : D'après les observations suivantes, une partie du produit (environ 15%) est sous forme énolique :
- 3,60 ppm, le singulet a une intégration inférieure à 2 H.
7,20 ppm : s : H éthylique de la forme énolique.
- les signaux de l'éthyle de l'ester éthylique sont légèrement déformés.

### H) [((dihydro-4,5 oxo-5 isoxazol-3-yl)-2' biphényl-4-yl) méthyl]-1 n-propyl-2 spirocyclohexane-4 imidazoline-2 one-5.

Ce composé est préparé selon la méthode décrite par F De Sarlo et al., Tetrahedron, 1966, 22, 2989-2994.

300 mg du produit obtenu à l'étape précédente sont dissous dans 3 ml de pyridine, on additionne 52 mg de chlorhydrate d'hydroxylamine et chauffe à 100°C pendant 2 heures 45 minutes. Le milieu réactionnel est repris par 100 ml d'acétate d'éthyle et 15 ml d'eau ; la phase organique est décantée, lavée par une solution saturée de chlorure de sodium, séchée sur sulfate de sodium puis concentrée sous vide, le résidu est chromatographié sur silice en éluant par un mélange acétate d'éthyle/méthanoVacide acétique (99,25/0,5/0,25 ; v/v/v). Après cristallisation par un mélange éther-hexane, on obtient 10 mg de produit.
Fc = 165-170°C avec décomposition.
Le produit est identifié par ses spectres.
IR (KBr)
1805 cm-¹ : C = O : isoxazolone.

- Spectre RMN (CDCI₃) :
   7,20-7,90 ppm : m : 8 H : aromatiques
   4,80 ppm : s : 2 H : CH₂-C₆H₄-
   3,05 ppm : s : 2 H : CH₂ de l'isoxazolone
   2,40 ppm : t : 2 H : CH₃-CH₂-CH₂
   1,40-2,00 ppm : m : 12 H : 10 H du cyclohexane et CH₃-CH₂-CH₂-
   1,00 ppm : t : 3 H : CH₃ (n-propyl)
- Spectre de masse : MH⁺ : 444.

### EXEMPLE 5

### [(n-butyl-2 oxo-5 spirocyclopentane-4) imidazolin-2-yl-1] méthyl-4' biphénylcarbonyle-2 thiosemicarbazide. (I : R₂ = H, R₁ = -CONH-NH-CS-NH₂, R₃ = n-C₄H₉, CR₄R₅ = cyclopentane, X = O).

Le n-butyl-2 spirocyclopentane-4 imidazoline-2 one-5 est décrit dans l'exemple 1 et 2, étape A.

### A) n-butyl-2 spirocyclopentane-4 (tert-butoxycarbonyl-2' biphényl-4-yl) méthyl-1 imidazoline-2 one-5.

970 mg du produit obtenu à l'exemple 1, étape A) sont dissous dans 10 ml de diméthylformamide. On ajoute 270 ml de méthylate de sodium et laisse sous agitation pendant 15 minutes à température ambiante. On additionne 2,08 g de bromométhyl-4 (tert-butoxycarbonyl-2') biphényle à la suspension puis, après 30 minutes, on chauffe à 40°C sous azote pendant 3 heures et demie. Le milieu réactionnel est repris par un mélange contenant 100 ml d'acétate d'éthyle, 10 ml d'eau et 1 ml de solution saturée de bicarbonate de sodium. La phase organique est lavée par une solution saturée de chlorure de sodium puis séchée sur sulfate de sodium et évaporée à sec. Le résidu est chromatographié sur gel de silice en éluant par un mélange acétate d'éthyle/toluène (1/2; v/v). On obtient 1,25 g du produit attendu qui cristallise.

Fc = 63-66°C.

### B) trifluoroacétate de n-butyl-2 [(carboxy-2' biphényl-4-yl) méthyl]-1 spirocyclopentane-4 imidazoline-2 one-5.

1,22 g de produit obtenu à l'étape précédente est agité pendant 40 minutes dans une solution contenant 6 ml de dichlorométhane et 8 ml de TFA. Après concentration sous vide, le résidu est repris dans de l'éther éthylique ; le précipité blanc formé est filtré, lavé à l'éther, puis séché sous vide. On obtient 1,15 g du produit attendu.

Fc = 176-178°C.
- Spectre RMN :
   0,78 ppm : t : 3 H : CH₃ (nBu)
   1,25 ppm : sext : 2 H : CH₃-CH₂
   1,50 ppm : quint : 2 H :CH₃-CH₂-CH₂
   1,75-2,00 ppm : m : 8 H : cyclopentane
   2,65 ppm : t : 2 H : CH₃-CH₂-CH₂-CH₂-
   4,83 ppm : s : 2 H : CH₂-C₆H₄-
   7,20-7,75 ppm : m : 8 H : aromatiques
- Spectre de masse : MH⁺ : 405

### C) [(n-butyl-2 oxo-5 spirocyclopentane-4) imidazolin-2-yl-1] méthyl-4'-biphényl-carbonyle-2 thiosemicarbazide.

On mélange 101 mg de l'acide préparé à l'étape précédente, 25 mg de thiosemicarbazide, 5 ml de diméthylformamide, 111 mg de BOP et de la DIPEA en quantité suffisante pour maintenir à pH 8. Après 3 heures sous agitation à température ambiante, on évapore, reprend par de l'acétate d'éthyle, lave à l'eau, au carbonate de sodium, à l'eau puis par une solution saturée de chlorure de sodium. Le solide obtenu est chromatographié sur silice en éluant par un mélange de dichlorométhane-méthanol (100/3 ; v/v).

Le produit obtenu concrétise dans l'éther isopropylique.

Fc = 124-126°C.
- Spectre RMN :
   0,75 ppm : t : 3 H : CH₃-CH₂-CH₂-CH₂-
   1,25 ppm : sext : 2 H : CH₃-CH₂-CH₂-CH₂-
   1,45 ppm : quint : 2 H : CH₃-CH₂-CH₂-CH₂-
   1,55-1,90 ppm : m : 8 H : cyclopentane
   2,3 ppm : t : 2 H : CH₃-CH₂-CH₂-CH₂-
   4,7 ppm : s : 2 H : N-CH₂-C₆H₄-
   6,7 et 7,85 ppm : 2 s.e., 2 fois H : -CS-NH₂
   7,15-7,8 ppm : m : 8 H : aromatiques
   9,3 et 10,0 ppm : 2s, 2 fois H : NH-NH

### EXEMPLE 6

### [((amino-2 thiadiazol-1,3,4-yl-5)-2' biphényl-4-yl) méthyl]-1 n-butyl-2 spirocyclopentane-4 imidazoline-2 one-5. (1 : R₂ = H,

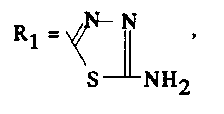

### R₃ = n-C₄H₉, CR₄R₅ = cyclopentane, X = O).

Ce composé est préparé en utilisant la méthode décrite par J. Sandstrom dans Advances in Heterocyclic Chemistry (ed. A.R. Katrizky et A.J. Boulton, Academic Press, 1968, 9, 175).

Dans 50 ml de toluène on mélange 350 mg du composé préparé à l'exemple précédent et 10 gouttes d'acide méthanesulfonique et on porte le mélange à reflux pendant 18 heures dans un ballon équipé d'un séparateur d'eau. On ajoute une solution de carbonate de sodium à 10% puis on extrait par un mélange acétate d'éthyle-toluène et l'on chromatographie sur colonne de silice en éluant par un mélange dichlorométhane-méthanol-acide acétique (98/1/1, v/v/v). Le produit obtenu est dissous dans de l'éther éthylique, lavé par une solution de carbonate de sodium à 10%, de l'eau, une solution saturée de chlorure de sodium.

On recueille 140 mg du produit attendu qui fond entre 90 et 100°C.
- Spectre RMN :
   0,9 ppm : t : 3 H : -CH₂-CH₂-CH₂-CH₃
   1,30 ppm : sext : 2 H : -CH₂-CH₂-CH₂-CH₃
   1,55 ppm : quint : 2 H :-CH₂-CH₂-CH₂-CH₃
   1,7-2 ppm : m : 8 H : cyclopentane
   2,4 ppm : t : 2 H : -CH₂-CH₂-CH₂-CH₃
   4,8 ppm : s : 2 H : N-CH₂-C₆H₄-
   7,2 ppm : 2 H : NH2
   7,2-7,9 ppm : m : 8 H : aromatiques

### EXEMPLE 7

### [(n-butyl-2 oxo-5 spirocyclopentane-4) imidazolin-2-yl-1] méthyl-4' biphénylcarbonyloxy-2 guanidine. (I : R₁ = COON=C (NH₂)₂, R₂ = H, R₃ = n-C₄H₉, CR₄R₅ = cyclopentane, X = O).

L'hémisulfate d'hydroxyguanidine est préparé selon J. Biol. Chem., 1959, 1481.

On agite à température ambiante pendant 18 heures un mélange contenant 1,036 g de trifluoroacétate de n-butyl-2 [(carboxy-2' biphényl-4-yl) méthyl]-1 spirocyclopentane-4 imidazoline-2-one-5 préparé à l'exemple 5, étape B), 496 mg d'hémisulfate d'hydroxy-2 guanidine, 50 ml de diméthylformamide, 888 mg de BOP et une quantité suffisante de DIPEA pour maintenir à pH 9-10. On évapore les solvants, reprend par de l'acétate d'éthyle, lave par de l'eau, une solution de carbonate de sodium, de l'eau, une solution saturée de chlorure de sodium. Le produit obtenu est purifié par chromatographie sur silice en éluant par un mélange dichlorométhaneméthanol (100/2,5 ; v/v). On obtient 285 mg du produit attendu sous forme de poudre amorphe qui fond entre 85°C et 95°C.
- Spectre RMN :
   0,9 ppm : t : 3 H : CH₃-CHₛ-CH₂-CH₂-
   1,35 ppm : sext : 2 H : CH₃-CH₂-CH₂-CH₂-
   1,6 ppm : quint : 2 H CH₃-CH₂-CH₂-CH₂-
   1,7-2 ppm : m : 8 H : cyclopentane
   2,4 ppm : t : 2 H : CH₃-CH₂-CH₂-C₋H₂-
   4,8 ppm : s : 2 H : N-CH₂-C₆H₄-
   4,9 ppm: s : 2H :NH₂
   5,1 ppm : s : 2 H : NH₂
   7,2-7,4 ppm : système AA', BB'(J = 8,5 Hz) 4 H : N-CH₂-C₆H₄
   7,4-7,9 ppm : m : 4 H : N-CH₂-C₆H₄-C₆H₄

### EXEMPLE 8

### (amino-3 oxadiazol-1,2,4-yl-5)-2' biphényl-4-yl méthyl)-1 n-butyl-2 spirocyclo-pentane-4 imidazoline-2 one-5. (I : R₂ = H,

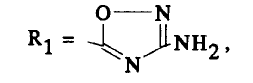

### R₃ = n-C₄H₉, CR₄R₅ = cyclopentane, X = O).

Ce composé est préparé en utilisant une méthode modifiée à partir de celle décrite par J. Saunders et al., J. Chem. Soc., Commun., 1988, 1618.

On place 340 mg du composé préparé à l'exemple précédent dans un tube à essai et on chauffe par un bain d'huile à 150°C pendant 1 heure. Après refroidissement, on reprend par du dichlorométhane et sèche sur sulfate de sodium puis on filtre et évapore. Le résidu obtenu est chromatographié sur silice en éluant par un mélange dichlorométhane- méthanol (98/2 ; v/v). On récupère 148 mg du produit attendu.

Fc = 156-158°C.
- Spectre RMN :
   0,9 ppm : t : 3 H : CH₃-CH₂-CH₂-CH₂-
   1,3 ppm : sext : 2 H : CH₃-CH₂-CH₂-CH₂-
   1,5 ppm : quint : 2 H : CH₃-CH₂-CH₂-CH₂-
   1,7-2 ppm : m : 8 H : cyclopentane
   2,4 ppm : t : 2 H : CH₃-CH₂-CH₂-CH₂-
   4,8 ppm : s : 2 H : N-CH₂-C₆H₄-
   6,3 ppm : s : 2H : NH₂
   7,2-7,35 ppm : système AA', BB', 4 H : N-CH₂-C₆H₄
   7,5-8 ppm : m : 4 H : N-CH₂-CₛH₄-C₆H₄

### EXEMPLE 9

### n-butyl-2 spirocyclopentane-4 [((triazol-1,2,4-yl-3)-2' biphényl-4-yl) méthyl]-1 imidazoline-2 one-5. (I : R₂ = H,

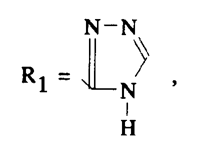

R₃ = n-C₄H₉, CR₄R₅ = cyclopentane, X = O).

### A) Préparation du bromométhyl-4' (trityl-triazol-1,2,4-yl-3)-2 biphényle.

### a) méthyl-4' biphénylcarbonyl-2 thiosemicarbazide.

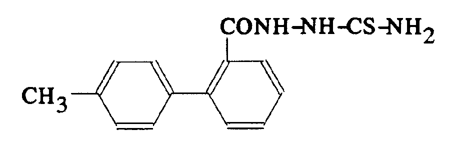

On place sous agitation pendant 1 journée à température ambiante un mélange contenant 6,4 g d'acide méthyl-4' biphényl-2 carboxylique, 3,0 g de thiosemicarbazide, 30 ml de diméthylformamide et 13,3 g de BOP et du DIPEA pour maintenir à pH basique (pH 8-9). On concentre, lave à l'eau, extrait par de l'acétate d'éthyle et isole 7,2 g du produit attendu.

### b) méthyl-4' [(mercapto-5) triazol-1,2,4-yl-3]-2 biphényle.

Ce composé est préparé selon la méthode décrite par H.L. Yale et al., J. Med. Chem., 1966, 9, 42.

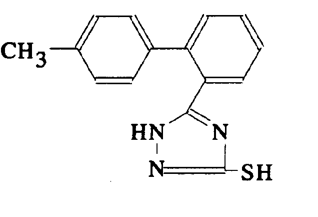

Dans 38 ml d'eau, on mélange 6,3 g du produit obtenu à l'étape Aa) et 1,16 g de soude et on chauffe à reflux pendant 1 heure. Après extraction à l'éther, on acidifie la phase aqueuse par de l'acide acétique, après essorage, on obtient le produit attendu sous forme d'un solide blanc.
m = 5,4 g
Fc = 250°C

### c) méthyl-4' [triazol-1,2,4-yl-3]-2 biphényle.

4,7 g du composé obtenu à l'étape précédente sont placés dans 100 ml d'éthanol en présence de Nickel de Raney et chauffés à reflux pendant 48 heures. On essore le milieu réactionnel puis on le concentre pour obtenir 4 g du produit attendu.

F = 146°C

### d) méthyl-4' [trityl-triazol-1,2,4-yl-3]-2 biphényle.

3,8 g du produit obtenu à l'étape précédente sont chauffés à reflux pendant 8 heures dans 100 ml de dichlorométhane en présence de 7,8 g de chlorure de trityle et 4,8 ml de triéthylamine. Après concentration du milieu réactionnel, on lave à l'eau et extrait à l'acétate d'éthyle. Le brut isolé est recristallisé dans l'hexane. On isole 7,7 g du produit attendu.

Fc = 120-122°C.

Remarque : la position de substitution du trityle n'a pas été déterminée.

### e) bromométhyl-4' [trityl-triazol-1,2,4-yl-3]-2 biphényle.

On chauffe à reflux pendant 3 heures 150 ml de tétrachlorure de carbone contenant 7,45 g du produit obtenu à l'étape précédente, 2,85 g de N-bromosuccinimide et 300 mg de peroxyde de benzoyle. On essore puis on concentre le milieu réactionnel pour obtenir 11,25 g du produit brut utilisé tel quel à l'étape suivante.

### B) Préparation du produit final.

### a) n-butyl-2 spirocyclopentane-4 [((trityl-triazol-1,2,4-yl-3)-2' biphényl-4-yl) méthyl]-1 imidazoline-2 one-5.

On agite à température ambiante pendant 30 minutes un mélange contenant 500 mg de l'imidazolinone préparée à l'exemple 1, étape A, 129 mg d'hydrure de sodium à 80%, 10 ml de diméthylformamide puis on ajoute 1,7 g du composé préparé à l'étape A du présent exemple et 10 ml de diméthylformamide. Après 5 heures sous agitation à température ambiante, on concentre puis on reprend le résidu par de l'acétate d'éthyle, lave à l'eau, sèche sur sulfate de sodium, filtre puis concentre sous pression réduite. On chromatographie le résidu sur silice en éluant par un mélange dichlorométhane-acétate d'éthyle (9/1 ; v/v). On obtient 700 mg du produit attendu.

### b) n-butyl-2 spirocyclopentane-4 [((triazol-1,2,4-yl-3)-2' biphényl-4-yl) méthyl]-1 imidazoline-2 one-5.

On laisse sous agitation à température ambiante pendant 6 heures, 700 mg du produit obtenu à l'étape Ba) dans 8 ml de méthanol et 0,6 ml d'acide chlorhydrique 4N. On concentre le milieu réactionnel puis reprend le résidu par de la soude 2N et extrait à l'éther. On acidifie la phase aqueuse par de l'acide chlorhydrique 1 N puis on extrait par de l'acétate d'éthyle.

Enfin on chromatographie sur silice en éluant par un mélange dichlorométhane/méthanol (97/3 ; v/v). On obtient 290 mg du produit attendu.

Fc = 87_{°}C
- Spectre RMN :
   0,7 ppm : t : 3 H : CH₃-CH₂-CH₂-CH₂-
   1,2 ppm : sext : 2 H : CH₃-CH₂-CH₂-CH₂-
   1,4 ppm : quint : 2 H :CH₃-CH₂-CH₂-CH₂-
   1,5-1,9 ppm : m : 8 H : cyclopentane
   2,2 ppm : t : 2 H : CH₃-CH₂-CH₂-CH₂-
   4,6 ppm : s : 2 H : N-CH₂-C₆H₄-
   6,9-7,1 ppm : système AA', BB', 4 H : N-CH₂-C₆H₄
   7,3-7,8 ppm : m : 4 H : N-CH₂-C₆H₄-C₆H₄
   8,1 ppm : s : 1 H : H (triazole).

### EXEMPLE 10

### [(n-butyl-2 oxo-5 spirocyclopentane)-4 imidazolin-2-yl-1] méthyl-4' biphénylcarbonyle-2 semicarbazide. (1 : R₁ = CONH-NH-CO-NH₂, R₂ = H, R₃ = n-C₄H₉, CR₄R₅ = cyclopentane, X = O).

L'imidazoline-2 one-5 préparée à l'exemple 5, étape B est utilisée sous forme de base libre ; 320 mg de ce composé sont mis en solution dans 7 ml de DMF et traités par 390 mg de BOP, 90 mg de chlorhydrate de semicarbazide et 272 µl de DIPEA. La réaction est agitée pendant 1 heure à température ambiante en maintenant à pH 7 par addition de DIPEA. On concentre le milieu réactionnel, l'huile obtenue est reprise par 100 ml d'acétate d'éthyle, lavée par 50 ml d'une solution saturée de chlorure de sodium, 2 fois par 20 ml d'une solution saturée d'hydrogénocarbonate de sodium puis par 20 ml de solution saturée de chlorure de sodium. On sèche sur sulfate de sodium puis filtre et concentre. Après recristallisation dans un mélange éther éthylique-hexane, on obtient 330 mg du produit attendu.
- Spectre RMN :
   0,9 ppm t : 3 H : CH₃ (nBu)
   1,4 ppm sext : 2 H : CH₂-CH₃
   1,6 ppm quint : 2 H : CH₂-CH₂₋CH₃
   1,7-2,00 ppm : m : 8 H : cyclopentane
   2,45 ppm : t : 2 H : CH₂-CH₂-CH₂-CH₃
   4,8 ppm : s : 2 H : CH₂-C₆H₄-
   5,95 ppm : s : 2 H : CONH₂
   8,0 ppm:s:1 H : CONH
   10,0 ppm : s : 1 H : CONH

### EXEMPLE 11

### n-butyl-2 [((oxo-3 oxadiazol-1,2,4-yl-5)-2' biphényl-4-yl) méthyl]-1 spirocyclopentane-4 imidazoline-2 one-5. (1 :

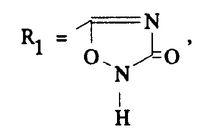

### R₂ = H, R₃ = n-C₄H₉, CR₄R₅ = cyclopentane, X = O).

### A) [(carbamoyl-2' biphényl-4-yl) méthyl]-1 n-butyl-2 spirocyclopentane-4 imidazoline-2 one-5.

L'imidazoline-2 one-5 préparée à l'exemple 5, étape B est utilisée sous forme de base libre. On dissout 2,65 g de ce composé dans 30 ml de dioxanne et 10 ml de DMF et l'on traite par 3,19 g de BOP et 1,24 ml de DIPEA pendant 1 heure à température ambiante. On ajoute à 0°C cette solution par portions, à une solution contenant 10 ml de dioxanne et 20 ml d'ammoniaque à 20% dans l'eau. On laisse 1 heure à 0°C, puis 2 heures à température ambiante. On concentre à sec, reprend par de l'acétate d'éthyle, un précipité de HOBT est éliminé. Le filtrat est lavé par de l'eau, une solution saturée d'hydrogénocarbonate de sodium, une solution saturée de chlorure de sodium, une solution KHSO₄-K₂SO₄, une solution de chlorure de sodium saturée. La phase organique est concentrée, reprise par un mélange éther éthylique-hexane. Le solide blanc obtenu est essoré et séché.
m = ₂,55 g
Fc = 115-120°C

### B) n-butyl-2 [(isocyanatocarbonyl-2' biphényl-4-yl) méthyl]-1 spirocyclopentane-4 imidazoline-2 one-5.

Ce composé est préparé selon A.J. Speziale, J. Org. Chem., 1965, 30, 4306.

(1 : R₁ = -CON=C=O, R₂ = H, R₃ = n-C₄H₉, CR₄R₅ = cyclopentane, X = O).

1 g de l'amide préparée à l'étape précédente est placé dans 15 ml de dichloro-1,2 éthane. On ajoute 300 µl de chlorure d'oxalyle à 0°C, puis on laisse revenir à température ambiante et on chauffe à reflux pendant 6 heures. Après concentration à sec, la mousse noire est utilisée telle quelle à l'étape suivante.

### C) n-butyl-2 [((oxo-3 oxadiazol-1,2,4-yl-5)-2' biphényl-4-yl) méthyl]-1 spirocyclopentane-4 imidazoline-2 one-5.

Le produit obtenu précédemment est traité par 1 ml d'azidure de triméthylsilyle dans 10 ml de xylène pendant 4 heures à 120°C et on concentre à sec. On ajoute 10 ml d'éthanol, concentre à sec après 30 minutes d'agitation, puis le résidu est trituré dans du méthanol. Le solide marron obtenu est essoré puis rincé par du méthanol, de l'éther éthylique et séché.
m = ₁₇₀ mg
Fc = 192-195°C

- Spectre de masse MH⁺ : 445
- Spectre RMN :
   0,85 ppm : t : 3 H : CH₃ (nBu)
   1,25 ppm : sext : 2 H : CH₃-CH₂
   1,6 ppm : quint : 2 H : CH₃-CH₂-CH₂
   1,7-2,0 ppm : m : 8 H : cyclopentane
   2,4 ppm : t : 2 H : CH₃-CH₂-CH₂-C₋H₂-
   4,8 ppm : s : 2 H : CH₂-C₆H₄-
   7,2-8,0 ppm : m : 8 H : aromatiques

### EXEMPLE 12

(R,S) n-butyl-2 cyclohexyl-4 méthyl-4 [((oxo-5 oxadiazol-1,2,4-yl-3)-2' biphényl-4-yl) méthyl]-1 imidazoline-2 one-5. (I : 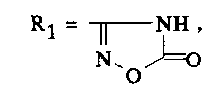 R₂ = H , R₃ = n-Bu, R₄ = méthyl, R₅ = cyclohexyl, X = 0 )

### A) Cyclohexyl-5 méthyl-5 hydantoïne.

Ce composé est préparé selon J. Org. Chem., 1960, 25, 1920-1924.

50 g de cyclohexylméthylcétone, dissous dans 400 ml d'alcool 95, sont ajoutés en 30 minutes à 29,4 g de cyanure de sodium et 192 g de carbonate d'ammonium dans 400 ml d'eau. On chauffe à 55-60°C pendant 4 heures puis on évapore de moitié sous vide et on laisse une nuit à +4°C. Le précipité formé est filtré, lavé à l'eau, puis séché sous vide sur anhydride phosphorique. On obtient 65,5 g de l'hydantoïne attendue, identifiée par ses spectres IR et RMN.

Fc = 220°C

### B) Acide (R,S) amino-2 cyclohexyl-2 propionique.

Ce composé est préparé selon J. Org. Chem. 1960, 25, 1920-1924.

Un mélange contenant 7 g de l'hydantoïne, préparée à l'étape précédente et 28 g d'octahydrate d'hydroxyde de baryum dans 150 ml d'eau, est porté à 160°C pendant 5 heures, dans un tube d'acier. Le milieu réactionnel est saturé avec de la carboglace, l'insoluble formé est filtré, puis le filtrat est concentré sous vide. Le résidu est repris dans de l'acétone, filtré et séché. On obtient 5,25 g de l'acide attendu, identifié par ses spectres IR et RMN. Le produit fond en se décomposant à 350°C.

### C) Ester éthylique de l'acide (R,S) amino-2 cyclohexyl-2 propionique.

3 g de l'acide préparé à l'étape précédente sont ajoutés à 40 ml d'alcool absolu saturé en gaz chlorhydrique, puis on chauffe à reflux, sous agitation, pendant 20 heures. Le milieu réactionnel est évaporé sous vide et le résidu est repris dans un mélange éther-eau que l'on amène à pH9 par addition d'une solution saturée de bicarbonate de sodium. La phase organique est décantée, lavée par une solution saturée de chlorure de sodium, puis évaporée sous vide. On obtient 2,1 g de l'ester attendu sous forme d'huile. Identification par spectres IR et RMN.

### D) Valérimidate d'éthyle.

Ce composé est préparé sous forme de chlorhydrate selon Mac Elvain (J. Amer. Chem. Soc., 1942, 64, 1825-1827). Il est libéré de son chlorhydrate par action du carbonate de potassium, puis extrait par le DCM.

### E) (R,S) n-butyl-2 cyclohexyl-4 méthyl-4 imidazoline-2 one-5.

2 g de l'ester préparé à l'étape C) et 2,35 g de valérimidate d'éthyle sont mélangés dans 6 ml de xylène auxquels on ajoute 6 gouttes d'acide acétique ; on porte à reflux pendant 6 heures. Le milieu réactionnel est ensuite concentré sous vide et le résidu est chromatographié sur gel de silice fine en éluant par un mélange chloroforme/méthanol/ acide acétique (95/9/3 ; v/v/v). Les fractions contenant le produit désiré sont rassemblées puis évaporées sous vide ; le résidu est repris par un mélange acétate d'é thyle/eau et on amène à pH9 par addition d'une solution de soude. La phase organique est décantée, lavée à l'eau, puis par une solution saturée de chlorure de sodium, séchée sur sulfate de sodium, puis évaporée à sec. On obtient le produit attendu sous forme d'une huile épaisse qui se prend en masse pour donner un solide amorphe.
m = 1,56 mg
   IR (chloroforme) :
1720 cm⁻¹ C=O
1640 cm-¹ C_{=N}
RMN conforme

### F) (R,S) [(n-butyl-2 cyclohexyl-4 méthyl-4 oxo-5) imidazolin-2-yl-1] méthyl-4' biphényl-2 (N-méthoxycarbonyl) carboximidate d'éthyle.

(I : R₂ = H, 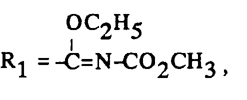 R₃ = n-C₄H₉, X = O, R₄ = méthyl, R₅ = cyclohexyl)

On met en suspension sous argon 270 mg d'hydrure de sodium à 80% dans l'huile et 10 ml de DMF anhydre et on ajoute par petites fractions à 0°C, 1,9 g du composé préparé à l'étape E). Après 20 minutes d'agitation à TA sous argon, on ajoute 5 g du composé préparé à l'exemple1-2, étape C), dissous dans 15 ml de DMF anhydre. Après 1 heure d'agitation à TA sous argon, on concentre le DMF sous vide et on reprend l'huile obtenue dans 100 ml d'acétate d'éthyle. On lave avec 50 ml d'eau, 50 ml de solution d'hydrogénosulfate de potassium à 5%, puis une solution saturée de chlorure de sodium et 50 ml de solution saturée d'hydrogénocarbonate de sodium. On sèche la phase organique, filtre et concentre. L'huile résiduelle est chromatographiée sur silice en éluant par un mélange heptane/AcOEt (8/2 ; v/v) pour obtenir le produit attendu;

m=₃,₁₀g g
spectre de masse : MH⁺ = 532
- Spectre RMN
   0,9 ppm : t : 3 H : CH₃ (nBu)
   1,0 ppm : t : 3 H : OCH₂-CH₃
   1,0-1,9 ppm : m : 18 H : cyclohexyl-4, CH₃-4, CH₂-CH₂-CH₂-CH₃ (nBu)
   2,4 ppm : t : 2 H : CH₂-C₂-CH₂-CH₃ (nBu)
   3,4 ppm : s : 2 H : CO₂-CH₃
   3,55 ppm : t : 2 H : OCH₂-CH₃
   4,7 ppm : s : 2 H : CH₂-C₆H₄-
   7,2-7,6 ppm : m : 8 H : aromatiques

### G) (R,S) n-butyl-2 cyclohexyl-4 méthyl-4 [((oxo-5 oxadiazol-1,2,4-yl-3)-2' biphényl-4-yl) méthyl]-1 imidazoline-2 one-5.

On place dans un ballon sous argon 354 mg de méthylate de sodium à 95% dans le méthanol et 432 mg de chlorhydrate d'hydroxylamine dans 25 ml de méthanol. On laisse sous agitation 15 minutes à TA puis on ajoute 3 g du composé obtenu à l'étape précédente dans 25 ml de méthanol. On porte à reflux sous argon pendant 15 heures en maintenant une forte agitation. On concentre le milieu réactionnel, reprend par 50 ml de DCM puis on extrait par 50 ml d'eau chlorhydrique (pH5) puis par 50 ml de solution de lessive de soude (pH13). On acidifie la phase alcaline à pH2 ; celle-ci est extraite 2 fois par 50 ml de DCM, puis la phase organique est séchée sur sulfate de sodium puis concentrée. Le résidu est chromatographié sur silice en éluant par un mélange heptane/AcOEt/AcOH (80/20/1,5). Le produit attendu est obtenu sous forme d'une huile qui cristallise après trituration dans un mélange éther/hexane.
m = 1 g
Fc = 210-215°C

- Spectre de masse : MH⁺ : 487
- Spectre RMN
   0,7 ppm : t : 3 H : CH₃ (nBu)
   0,8-1,8 ppm : m : 18 H : cyclohexyl-4, méthyl-4, CH₂-CH₂-CH₂-CH₃ (nBu)
   2,25 ppm : t : 2 H : CH₃-CH₂-CH₂-CH₂- (nBu)
   4,6 ppm : s : 2 H : CH₂-C₆H₄-
   7,1-7,6 ppm : m : 8 H : aromatiques
   12,3 ppm : s : 1 H : NH

### EXEMPLE 13

### n-butyl-2 cyclohexyl-4 méthyl-4 [((oxo-5 oxadiazol-1,2,4-yl-3)-2' biphényl-4-yl) méthyl]-1 imidazoline-2 one-5, dextrogyre.

### A) méthyl-5 phényl-5 hydantoïne.

30 g d'acétophénone, dilués dans 250 ml d'alcool 95, sont ajoutés en 30 minutes à un mélange de 18,35 g de cyanure de sodium et 125 g de carbonate d'ammonium dans 250 ml d'eau et l'on chauffe à 60-65°C, sous agitation, pendant 22 heures. Le milieu réactionnel est concentré sous vide de moitié et le solide qui a précipité est filtré, lavé à l'eau, à l'éther, puis séché sous vide. On obtient 38 g de solide blanc, identifié par IR.

Fc = 190-192°C

### B) Acide (R,S) amino-2 phényl propionique.

20 g du composé préparé à l'étape précédente sont ajoutés à un mélange de 75 g d'octahydrate d'hydroxyde de baryum dans 500 ml d'eau, puis on chauffe à 160°C pendant 5 heures dans un tube d'acier. Le milieu réactionnel est saturé de carboglace, puis le précipité est filtré. Le filtrat est concentré sous vide, le solide blanc formé est repris dans de l'acétone, filtré, lavé à l'acétone, à l'éther, puis séché. On obtient 15,3 g de l'acide attendu.

Fc = 260-265°C (avec décomposition).

### C) Ester éthylique de l'acide (R,S) amino-2 phényl-2 propionique.

A une solution de 80 g de gaz chlorhydrique dans 210 ml d'éthanol absolu, on ajoute à la spatule, sous agitation, 24 g d'acide préparé à l'étape précédente et l'on chauffe à reflux pendant 6 heures et demie. On concentre sous vide, on reprend le résidu dans 600 ml d'acétate d'éthyle et 100 ml d'eau et l'on ajoute de la soude 2N jusqu'à pH9. La phase organique est décantée, lavée à l'eau, par une solution saturée de chlorure de sodium, séchée sur sulfate de sodium puis concentrée sous vide. On obtient 25,5 g du produit attendu sous forme d'huile. identification par IR.

### D) Ester éthylique dextrogyre de l'acide amino-2 phényl-2 propionique.

On sépare les énantiomères de l'ester préparé à l'étape précédente par la méthode décrite par Y Sugi et S. Mitsui dans Bull. Chem. Soc. Japan, 1969, 42, 2984-2988. Aux 25,5 g d'ester obtenus à l'étape C), dilués dans 210 ml d'éthanol absolu, on ajoute 19,8 g d'acide (L) (+) tartrique. On chauffe à 60°C pour tout dissoudre puis on laisse à TA pendant 4 heures. Les cristaux formés sont filtrés puis rincés par 2 fois 70 ml d'alcool absolu, puis ils sont redissous dans 200 ml d'alcool à ébullition puis on laisse à TA pendant 72 heures. Les cristaux en aiguille formés sont filtrés, rincés 2 fois par 30 ml d'alcool puis séchés sous vide. On obtient 11,9 g du sel d'acide tartrique de l'ester dextrogyre attendu.

Fc = 172-173°C
[α]_{D} = + 44,5)° (C=1, eau)

La solution alcoolique restante est enrichie en sel d'acide tartrique de l'ester lévogyre.

6,1 g du sel d'acide tartrique de l'ester dextrogyre sont repris dans 30 ml d'eau puis 200 ml d'acétate d'éthyle et l'on ajoute de la soude 5N jusqu'à pH9. La phase organique est décantée, lavée à l'eau, par une solution saturée de chlorure de sodium, séchée sur sulfate de sodium puis concentrée sous vide. On obtient 3,33 g du produit attendu sous forme d'huile.

[a]p = + 24° (C=₂, éthanol) Identification par RMN.

### E) ester éthylique lévogyre de l'acide amino-2 phényl-2 propionique.

On concentre sous vide la solution alcoolique obtenue à l'étape précédente, après la séparation des cristaux de sel d'acide tartrique de l'ester éthylique dextrogyre de l'acide amino-2 phényl-2 propionique. Le résidu solide est repris dans 150 ml d'eau et 600 ml d'acétate d'éthyle et on amène à pH 9 par addition de soude 5N. La phase organique est décantée, lavée à l'eau, par une solution saturée de chlorure de sodium, séchée sur sulfate de sodium puis concentrée sous vide. On obtient 20,6 g d'ester enrichi en levogyre.

A 20,5 g de cet ester, dilués dans 200 ml d'éthanol absolu, on ajoute 15,9 g d'acide D(-) tartrique et on dissout à ébullition de l'alcool. Après 5 heures à TA, les cristaux en aiguille formés sont filtrés, lavés 2 fois par 50 ml d'alcool absolu puis séchés sous vide. On obtient 16,3 g de sel tartrique du produit attendu.
Fc = 172-173°C
[α]D = - 45,2° (C=1, eau)

6 g du sel obtenu sont repris dans 50 ml d'eau et 200 ml d'acétate d'éthyle et on amène à pH9,5 par addition de soude 5N. La phase organique est décantée, lavée à l'eau, par une solution saturée de chlorure de sodium, puis séchée sur sulfate de sodium et concentrée sous vide. On obtient 3,31 g du produit attendu sous forme d'huile; identifiable par RMN.
[α]D = - 25,5° (C=1, éthanol)

### F) ester éthylique lévogyre de l'acide amino-2 cyclohexyl-2 propionique.

3,30 g de l'ester levogyre, obtenu à l'étape précédente, sont dilués dans 120 ml d'acide acétique ; on ajoute 1,5 g d'oxyde de platine puis on hydrogène à pression atmosphérique. Après 40 heures d'hydrogénation, le milieu réactionnel est filtré puis concentré sous vide. Le résidu est repris dans un mélange éther-eau et l'on ajoute de l'acide chlorhydrique 6N, jusqu'à pH2. La phase aqueuse est décantée puis on ajoute de l'acétate d'éthyle puis de la soude 5N, jusqu'à pH 9,5. La phase organique est décantée, lavée à l'eau, par une solution de chlorure de sodium saturée, séchée sur sulfate de sodium, puis concentrée sous vide. On obtient 3,20 g du produit attendu.

[α]p = - 19,2° (C=₁, éthanol). Littérature : WA. Bonner et al., J Amer. Chem. Soc., 1956, 78, 3218-3221.

Identification par RMN.

### G) n-butyl-2 cyclohexyl-4 méthyl-4 imidazoline-2 one-5, dextrogyre.

On porte à reflux sous agitation un mélange contenant 3 g d'ester levogyre obtenu à l'étape précédente, 4,7 g de valérimidate d'éthyle et 8 gouttes d'acide acétique dans 15 ml de xylène.

Après 7 heures de reflux, le milieu réactionnel est concentré sous vide. Le résidu est chromatographié sur silice en éluant par un mélange chloroforme/méthanol/acide acétique (95/9/3), les fractions contenant le produit sont rassemblées et concentrées sous vide. Le résidu est repris dans un mélange acétate d'éthyle/eau et on amène à pH 9 par addition de soude 5N. La phase organique est décantée, lavée à l'eau, par une solution saturée de chlorure de sodium, séchée sur sulfate de sodium et concentrée sous vide. On obtient une huile qui se transforme en solide amorphe.
m = ₂,36 g
[α]p = + 56,9° (c=1, chloroforme)
IR (chloroforme)
1720 cm⁻¹C=O
1640 cm-¹ C_{=N}

Le spectre IR confirme la forme one-5 de l'imidazolinone en solution.

### H) n-butyl-2 cyclohexyl-4 méthyl-4 [((oxo-5 oxadiazol-1,2,4-yl-3)-2' biphényl-4-yl) méthyl]-1 imidazoline-2 one-5, dextrogyre.

On procède ensuite comme à l'exemple 12, étapes F) et G) et l'on obtient le produit attendu.

[α]D = + 30,1° (c=1, méthanol)

Le spectre RMN est superposable à celui de l'exemple 12.

## Revendications (Revendications pour l'(les) Etat(s) contractant(s) suivant(s) : AT, BE, CH, DE, DK, FR, GB, IT, LI, LU, NL, PT, SE)

1. Un composé de formulé : 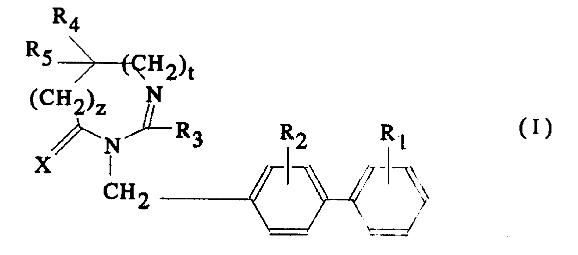 dans laquelle :
- R₁ est en position ortho et représente un hétérocycle azoté choisi parmi : 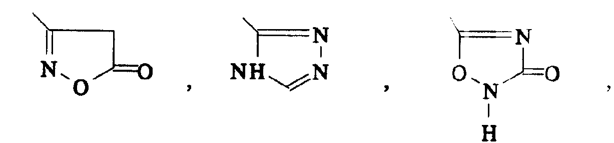 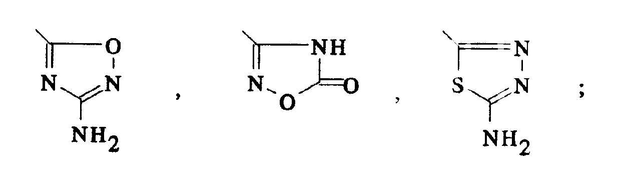
- R₂ est l'hydrogène ;
- R₃ représente un alkyle en C₁-C₆ ;
- R₄ et R₅ liés ensemble avec le carbone auquel ils sont attachés, représentent un groupe de formule (CH₂)ₙ ;
- n est égal 4 ou 5 ;
- z et t sont nuls ou z représente 1 et t est nul ;
- X représente un atome d'oxygène ;
et ses sels.

2. Un composé selon la revendication 1, choisi parmi :
- la n-butyl-2 [((oxo-5 oxadiazol-1,2,4-yl-3)-2' biphényl-4-yl) méthyl]-1 spirocyclopentane-4 imidazoline-2 one-5 ;
- la [((dihydro-4,5 oxo-5 isoxazol-3-yl)-2' biphényl-4-yl) méthyl]-1 n-propyl-2 spirocyclohexane-4 imidazoline-2 one-5 ;
- la [((amino-2 thiadiazol-1,3,4-yl-5)-2' biphényl-4-yl) méthyl]-1 n-butyl-2 spirocyclopentane-4 imidazoline-2 one-5;
- la [((amino-3 oxadiazol-1,2,4-yl-5)-2' biphényl-4-yl) méthyl]-1 n-butyl-2 spirocyclopentane-4 imidazoline-2 one-5;
- la n-butyl-2 spirocyclopentane-4 [((triazol-1,2,4-yl-3)-2' biphényl-4-yl) méthyl]-1 imidazoline-2 one-5 ; ou
- la n-butyl-2 [((oxo-3 oxadiazol-1,2,4-yl-5)-2' biphényl-4-yl) méthyl]-1 spirocyclopentane-4 imidazoline-2 one-5.

3. Un composé choisi parmi :
- le [(n-butyl-2 oxo-5 spirocyclopentane-4) imidazolin-2-yl-1] méthyl-4' biphényl-2-yl- (N-méthoxycarbonyl) carboximidate d'éthyle ;
- le [((n-propyl-2 oxo-5 spirocyclohexane-4) imidazolin-2-yl-1) méthyl-4' biphényl-2-yl]-3 oxo-3 propionate d'éthyle ;
- le [(n-butyl-2 oxo-5 spirocyclohexane-4) imidazolin-2-yl-1] méthyl-4' biphényl-carbonyle-2 thiosemicarbazide ;
- la [(n-butyl-2 oxo-5 spirocyclopentane-4) imidazolin-2-yl-1] méthyl-4'biphényl-carbonyloxy-2 guanidine ;
- le [(n-butyl-2 oxo-5 spirocyclopentane-4) imidazolin-2-yl-1] méthyl-4' biphényl-carbonyle-2 semicarbazide ;
- la (R,S) n-butyl-2 cyclohexyl-4 méthyl-4 [((oxo-5 oxadiazol-1,2,4-yl-3)-2' biphényl-4-yl) méthyl]-1 imidazoline-2 one-5 ;
- la n-butyl-2 cyclohexyl-4 méthyl-4 [((oxo-5 oxadiazol-1,2,4-yl-3)-2' biphényl-4-yl) méthyl]-1 imidazoline-2 one-5, dextrogyre.

4. Procédé pour la préperation d'un composé (I) selon la revendication 1 caractérisé en ce que :
a) on fait réagir un dérivé hétérocyclique de formule : 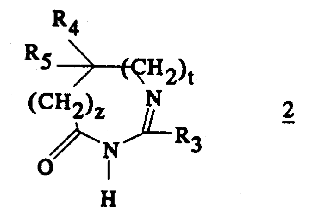 dans laquelle, z, t, R₃, R₄ et R₅ ont les significations indiquées pour (1) dans la revendication 1, sur a dérivé du (biphényl-4-yl) méthyle : 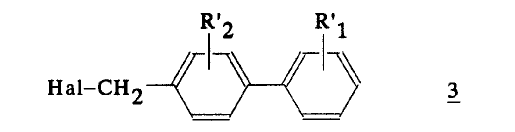 dans laquelle Hal représente un atome d'halogène et R'₁ et R'₂ représentent respectivement soit R₁ et R₂, soit un précurseur de R₁ et/ou R₂ ;
b) le composé ainsi obtenu, de formule : 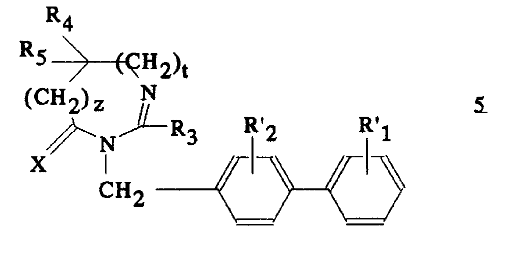 dans laquelle X représente un atome d'oxygène, est traité pour préparer le composé (I) par transformation des groupes R'₁ et/ou R'₂ on respectivement R₁ et/ou R₂.

5. Composition pharmaceutique contenant à titre de principe actif un composé selon l'une quelconque des revendications 1 à 3.

6. Composition pharmaceutique contenant un composé selon l'une quelconque des revendications 1 à 3 en association avec un composé bêtabloquant.

7. Composition pharmaceutique contenant un composé selon l'une quelconque des revendications 1 à 3 en association avec un diurétique.

8. Composition pharmaceutique contenant un composé selon l'une quelconque des revendications 1 à 3 en association avec un antiinflammatoire non stéroïdien.

9. Composition pharmaceutique contenant un composé selon l'une quelconque des revendications 1 à 3 en association avec un antagoniste calcique.

10. Composition pharmaceutique contenant un composé selon l'une quelconque des revendications 1 à 3 en association avec un tranquillisant.

## Revendications (Revendications pour l'(les) Etat(s) contractant(s) suivant(s) : ES)

1. Procédé pour l'obtention de composés de formule : 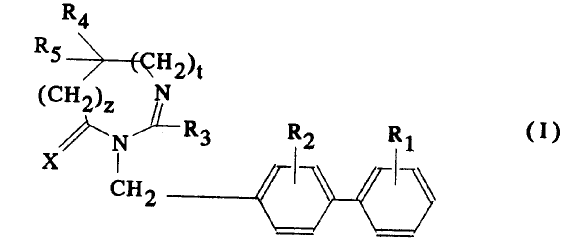 dans laquelle :
- Rᵢ est en position ortho et représente un hétérocycle azoté choisi parmi : 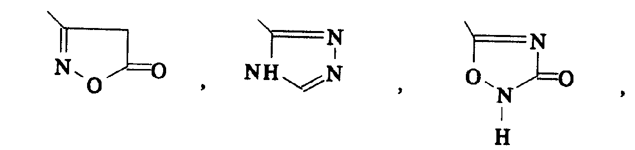 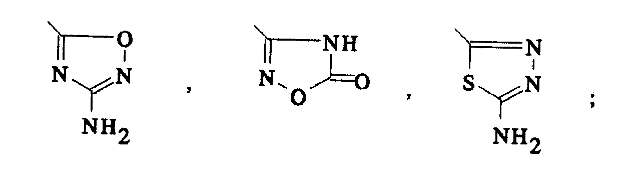
- R₂ est l'hydrogène ;
- R₃ représente un alkyle en C₁-C₆ ;
- R₄ et R₅ liés ensemble avec le carbone auquel ils sont attachés, représentent un groupe de formule (CH₂)ₙ ;
- n est égal à 4 ou 5;
- z et t sont nuls ou z représente 1 et t est nul ;
- X représente un atome d'oxygène ;
et leurs sels, caractérisé en ce que :
a) on fait réagir un dérivé hétérocyclique de formule : 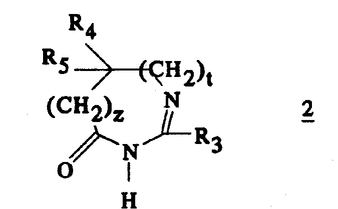 dans laquelle, z, t, R₃, R₄ et R₅ ont les significations indiquées ci-dessus, sur un dérivé du (biphényl-4-yl) méthyle : 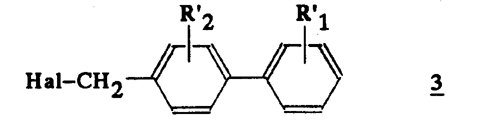 dans laquelle Hal représente un atome d'halogène et R'₁ et R'₂ représentent respectivement soit R₁ et R₂, soit un précurseur de R₁ et/ou R₂ ;
b) le composé ainsi obtenu, de formule : 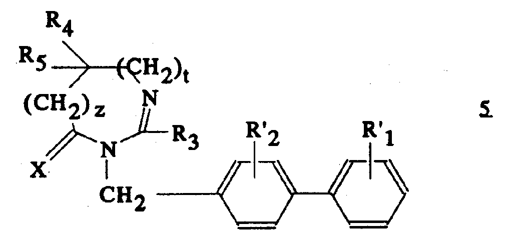 dans laquelle X représente un atome d'oxygène, est traité pour préparer le composé (I) par transformation des groupes R'₁ et/ou R'₂ en respectivement R₁ et/ou R₂.

2. Procédé selon la revendication 1, pour la préparation d'un composé choisi parmi :
- la n-butyl-2 [((oxo-5 oxadiazol-1,2,4-yl-3)-2' biphényl-4-yl) méthyl]-1 spirocyclopentane-4 imidazoline-2 one-5 ;
- la [((dihydro-4,5 oxo-5 isoxazol-3-yl)-2' biphényl-4-yl) méthyl]-1 n-propyl-2 spirocyclohexane-4 imidazoline-2 one-5 ;
- la [((amino-2 thiadiazol-1,3,4-yl-5)-2' biphényl-4-yl) méthyl]-1-n-butyl-2 spirocyclopentane-4 imidazoline-2 one-5;
la [((amino-3 oxadiazol-1,2,4-yl-5)-2' biphényl-4-yl) méthyl]-1 n-butyl-2 spirocyclopentane-4 imidazoline-2 one-5;
- la n-butyl-2 spirocyclohexane-4 [((triazol-1,2,4-yl-3)-2' biphényl-4-yl) méthyl]-1 imidazoline-2 one-5 ; ou la n-butyl-2 [((oxo-3 oxadiazol-1,2,4-yl-5)-2' biphényl-4-yl) méthyl]-1 spirocyclopentane-4 imidazoline-2 one-5 ;
caractérisé en ce qu'on utilise
(i) un dérivé 2 dans lequel z = t = 0 ; et R₃ représente un n-propyle et CR₄R₅ un cyclohexane ou le cas échéant R₃ représente un n-butyle et CR₄R₅ un cyclopentane ; et
(ii) un dérivé 3 dans lequel R'₂ = H et
* R'ᵢ = -C(OC₂H₅) = NC0₂CH₃ lorsqu'on désire obtenir un composé (I) pour lequel R₁ = 5-oxo-1,2,4-oxadiazol-3-yl ;
* R'₁ = t-butoxycarbonyl lorsqu'on désire obtenir un composé (I) pour lequel R₁ = 4,5-dihydro-5-oxo isoxa- zol-3-yl ; 2-amino-1,3,4-thiadiazol-5-yl ; 3-amino-1,2,4-oxadiazol-5-yl ou 3-oxo-1,2,4-oxadiazol-5-yl ;
* R'₁ = trityl-1,2,4-triazol-5-yl lorsqu'on désire obtenir un composé (I) pour lequel R₁ = 1,2,4-triazol-5-yl ;

3. Procédé pour la préparation d'un composé choisi parmi :
- le [(n-butyl-2 oxo-5 spirocyclopentane-4) imidazolin-2-yl-1] méthyl-4' biphényl-2-yl- (N-méthoxycarbonyl) carboximidate d'éthyle ;
- le [((n-propyl-2 oxo-5 spirocyclohexane-4) imidazolin-2-yl-1) méthyl-4' biphényl-2-yl]-3 oxo-3 propionate d'éthyle ;
- le [(n-butyl-2 oxo-5 spirocyclopentane-4) imidazolin-2-yl-1] méthyl-4' biphényl-carbonyle-2 thiosemicarbazide ;
- la [(n-butyl-2 oxo-5 spirocyclopentane-4) imidazolin-2-yl-1] méthyl-4' biphényl-carbonyloxy-2 guanidine ;
- le [(n-butyl-2 oxo-5 spirocyclopentane-4) imidazolin-2-yl-1] méthyl-4' biphényl-carbonyle-2 semicarbazide ;
- la (R,S) n-butyl-2 cyclohexyl-4 méthyl-4 [((oxo-5 oxadiazol-1,2,4-yl-3)-2' biphényl-4-yl) méthyl]-1 imidazoline-2 one-5; ou
- la n-butyl-2 cyclohexyl-4 méthyl-4 [((oxo-5 oxadiazol-1,2,4-yl-3)-2' biphényl-4-yl) méthyl]-1 imidazoline-2 one-5, dextrogyre,
caractérisé en ce que :
a) on fait réagir un dérivé hétérocylique de formule : 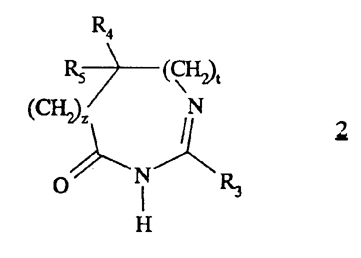 dans laquelle
- z = t = 0;
- R₃ représente un n-propyle et CR₄R₅ un cyclohexane ou le cas échéant R₃ représente un n-butyle et CR₄R₅ un cyclopentane ou bien R₃ représente un n-butyle, R₄ un méthyle et R₅ un cyclohexyle ;
sur un dérivé du (biphényl-4-yl)méthyle : 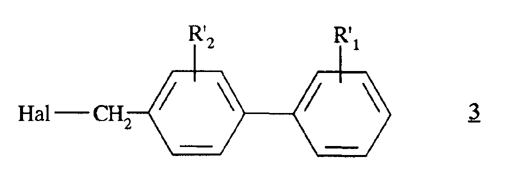 dans laquelle Hal représente un atome d'halogène, R'₂ = H et
* R'₁ = -C(OC₂H₅) = NC0₂CH₃ lorsqu'on désire obtenir un composé (I) pour lequel R₁ représente ce groupe ou un 5-oxo-1,2,4-oxadiazol-3-yl ;
^{*} R'₁ = t-butoxycarbonyl lorsqu'on désire obtenir un composé (I) pour lequel R₁ = -COCH₂CO₂C₂H₅ ; - CONHNHCSNH₂ ; -COON = C(NH₂)₂ ou -CONHNHCONH₂ ;
b) le composé ainsi obtenu, de formule: 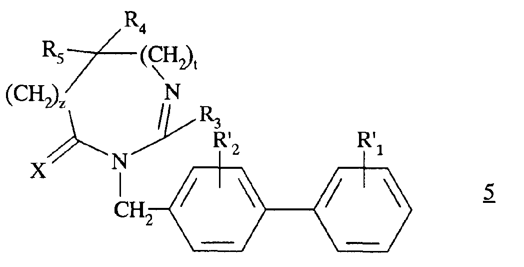 dans laquelle X représente un atome d'oxygène est traité pour préparer le composé (1) par transformation des groupes R'₁ et/ou R'₂ en respectivement R₁ et/ou R₂.

4. Procédé pour la préparation de compositions pharmaceutiques contenant à titre de principe actif un composé préparé selon le procédé de l'une quelconque des revendications 1 à 3, caractérisé en ce qu'on mélange ledit principe actif avec un véhicule pharmaceutiquement acceptable.

5. Procédé selon la revendication 4, caractérisé en ce que l'on utilise le principe actif en association avec un composé bêtabloquant

6. Procédé selon la revendication 4, caractérisé en ce que l'on utilise le principe actif en association avec un diurétique.

7. Procédé selon la revendication 4, caractérisé en ce que l'on utilise le principe actif en association avec un antiinflammatoire non stéroïdien.

8. Procédé selon la revendication 4, caractérisé en ce que l'on utilise le principe actif en association avec un antagoniste calcique.

9. Procédé selon la revendication 4, caractérisé en ce que l'on utilise le principe actif en association avec un tranquillisant.

## Revendications (Revendications pour l'(les) Etat(s) contractant(s) suivant(s) : GR)

1. Un composé de formule : 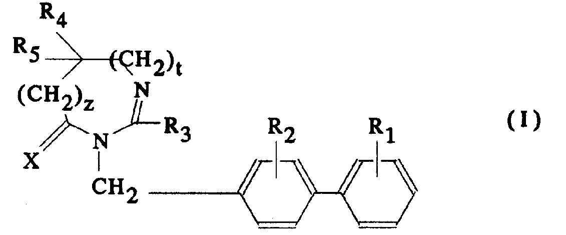 dans laquelle :
- R₁ est en position ortho et représente un hétérocycle azoté choisi parmi : 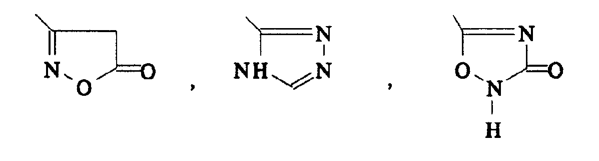 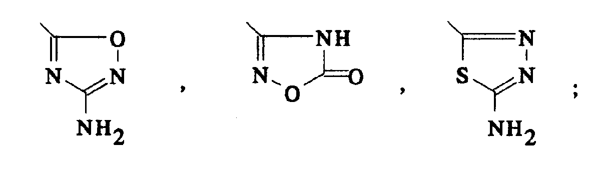
- R₂ est l'hydrogène ;
- R₃ représente un alkyle en C₁-C₆ ;
R₄ et R₅ liés ensemble avec le carbone auquel ils sont attachés, représentent un groupe de formule (CH₂)ₙ ;
- n est égal à 4 ou 5 ;
- z et t sont nuls ou z représente 1 et t est nul ;
X représente un atome d'oxygène ;
et ses sels.

2. Un composé selon la revendication 1, choisi parmi :
- la n-butyl-2 [((oxo-5 oxadiazol-1,2,4-yl-3)-2' biphényl-4-yl) méthyl]-1 spirocyclopentane-4 imidazoline-2 one-5 ;
- la [((dihydro-4,5 oxo-5 isoxazol-3-yl)-2' biphényl-4-yl) méthyl]-1 n-propyl-2 spirocyclohexane-4 imidazoline-2 one-5 ;
- la [((amino-2 thiadiazol-1,3,4-yl-5)-2 'biphényl-4-yl) méthyl]-1 n-butyl-2 spirocyclopentane-4 imidazoline-2 one-5;
- la [((amino-3 oxadiazol-1,2,4-yl-5)-2' biphényl-4-yl) méthyl]-1 n-butyl-2 spirocyclopentane-4 imidazoline-2 one-5;
- la n-butyl-2 spirocyclopentane-4 [((triazol-1,2,4-yl-3)-2' biphényl-4-yl) méthyl]-1 imidazoline-2 one-5; ou
- la n-butyl-2 [((oxo-3 oxadiazol-1,2,4-yl-5)-2' biphényl-4-yl) méthyl]-1 spirocyclopentane-4 imidazoline-2 one-5;

3. Un composé choisi parmi:
le [(n-butyl-2 oxo-5 spirocyclopentane-4) imidazolin-2-yl-1] méthyl-4' biphényl-2-yl-(N-méthoxycarbonyl)carboximidate d'éthyle;
- le [((n-propyl-2 oxo-5 spirocyclohexane-4) imidazolin-2-yl-1) méthyl-4' biphényl-2-yl]-3 oxo-3 propionate d'éthyle;
- le [(n-butyl-2 oxo-5 spirocyclopentane-4) imidazolin-2-yl-1] méthyl-4' biphényl-carbonyle-2 thiosemicarbazide;
la [(n-butyl-2 oxo-5 spirocyclopentane-4) imidazolin-2-yl-1] méthyl-4' biphényl-carbonyloxy-2 guanidine;
- le [(n-butyl-2 oxo-5 spirocyclopentane-4) imidazolin-2-yl-1] méthyl-4' biphényl-carbonyle-2 semicarbazide;
- la (R,S) n-butyl-2 cyclohexyl-4 méthyl-4 [((oxo-5 oxadiazol-1,2,4-yl-3)-2' biphényl-4-yl) méthyl]-1 imidazoline-2 one-5; ou
- la n-butyl-2 cyclohexyl-4 méthyl-4 [((oxo-5 oxadiazol-1,2,4-yl-3)-2' biphényl-4-yl) méthyl]-1 imidazoline-2 one-5, dextrogyre.

4. Procédé pour la préparation d'un composé (I) selon la revendication 1 caractérisé en ce que:
a) on fait réagir un dérivé hétérocyclique de formule: 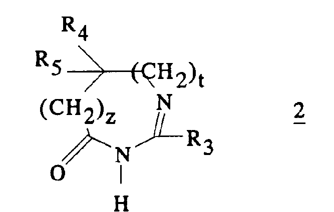 dans laquelle, z, t, R₃, R₄ et R₅ ont les significations indiquées pour (I) dans la revendication 1, sur un dérivé du (biphényl-4-yl) méthyle: 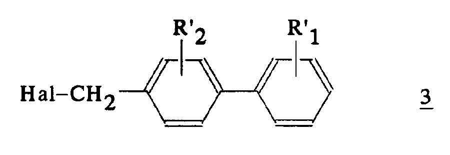 dans laquelle Hal représente un atome d'halogène et R'₁ et R'₂ représentent respectivement soit R₁ et R₂, soit un précurseur de R₁ et/ou R₂ ;
b) le composé ainsi obtenu, de formule : 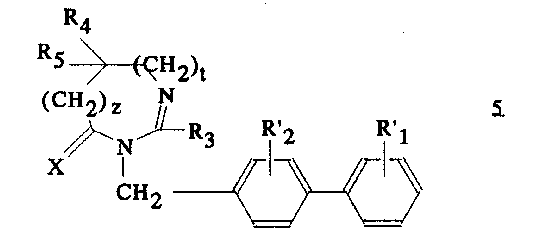 dans laquelle X représente a atome d'oxygène, est traité pour préparer le composé (I) par transformation des groupes R'₁ et/ou R'₂ en respectivement R₁ et/ou R₂.

5. Procédé pour la préparation d'une composition pharmaceutique contenant à titre de principe actif un composé selon l'une quelconque des revendications 1 à 3, caractérisé en ce qu'on mélange ledit principe actif avec a véhicule pharmaceutiquement acceptable.

6. Procédé selon la revendication 5, caractérisé en ce qu'on utilise le principe actif en association avec un composé bêtabloquant.

7. Procédé selon la revendication 5, caractérisé en ce qu'on utilise le principe actif en association avec un diurétique.

8. Procédé selon la revendication 5, caractérisé en ce qu'on utilise le principe actif en association avec un antiinflammatoire non stéroïdien.

9. Procédé selon la revendication 5, caractérisé en ce qu'on utilise le principe actif en association avec un antagoniste calcique.

10. Procédé selon la revendication 5, caractérisé en ce qu'on utilise le principe actif en association avec un tranquillisant.

## Claims (Claims for the following Contracting State(s) : AT, BE, CH, DE, DK, FR, GB, IT, LI, LU, NL, PT, SE)

1. A compound of the formula 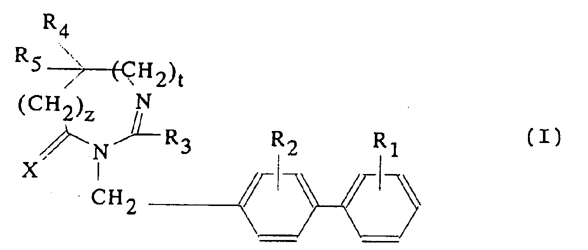 in which:
- R₁ is in the ortho position and is a nitrogen heterocycle selected from: 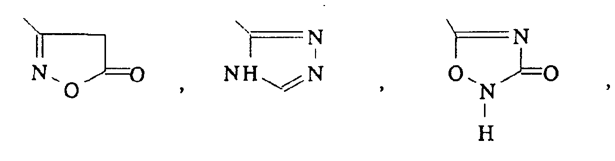 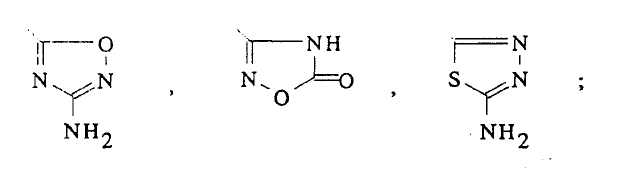
R₂ is hydrogen;
- R₃ is a C₁-C₆ alkyl;
- R₄ and R₅, bonded together with the carbon to which they are attached, are a group of the formula (CH₂)ₙ;
n is equal to 4 or 5;
- z and t are zero or z is 1 and t is zero; and
X is an oxygen atom;
and its salts.

2. A compound according to claim 1 which is selected from:
- 2-n-butyl-1-[(2'-(5-oxo-1,2,4-oxadiazol-3-yl)biphenyl-4-yl)methyl]-4-spirocyclopentane-2-imidazolin-5-one;
1-[(2'-(4,5-dihydro-5-oxoisoxazol-3-yl)biphenyl-4-yl)methyl]-2-n-propyl-4-spirocyclohexane-2-imidazolin-5- one;
- 1-[(2'-(2-amino-1,3,4-thiadiazol-5-yl)biphenyl-4-yl)-methyl]-2-n-butyl-4-spirocyclopentane-2-imidazolin-5-one;
1-[(2'-(3-amino-1,2,4-oxadiazol-5-yl)biphenyl-4-yl)-methyl]-2-n-butyl-4-spirocyclopentane-2-imidazolin-5-one;
- 2-n-butyl-4-spirocyclopentane-1 -[(2'-(1,2,4-triazol-3-yl)biphenyl-4-yl)methyl]-2-imidazolin-5-one; or
- 2-n-butyl-1-[(2'-(3-oxo-1,2,4-oxadiazol-5-yl)biphenyl-4-yl)methyl]-4-spirocyclopentane-2-imidazolin-5-one.

3. A compound selected from:
- ethyl 4'-[(2-n-butyl-5-oxo-4-spirocyclopentane)-2-imidazolin-1-yl]methylbiphenyl-2-yl-(N-methoxycarbonyl)-carboximidate;
- ethyl 3-[(4'-(2-n-propyl-5-oxo-4-spirocycolhexane)-2-imidazolin-1-yl)methylbiphenyl-2-yl]-3-oxopropionate;
- 4'-[(2-n-butyl-5-oxo-4-spirocyclopentane)-2-imidazolin-1-yl]methylbiphenyl-2-carbonylthiosemicarbazide;
- 4'-[(2-n-butyl-5-oxo-4-spirocyclopentane)-2-imidazolin-1-yl]methylbiphenyl-2-carbonyloxyguanidine;
- 4'-[(2-n-butyl-5-oxo-4-spirocyclopentane)-2-imidazolin-1-yl]methylbiphenyl-2-carbonylsemicarbazide; (R,S)-2-n-butyl-4-cyclohexyl-4-methyl-1-[(2'-(5-oxo-1,2,4-oxadiazol-3-yl)biphenyl-4-yl)methyl]-2-imidazolin-5- one; or
- 2-n-butyl-4-cyclohexyl-4-methyl-1-[(2'-(5-oxo-1,2,4-oxadiazol-3-yl)biphenyl-4-yl)methyl]-2-imidazolin-5-one, dextrorotatory

4. A method of preparing a compound (I) according to claim 1, characterized in that:
a) a heterocyclic derivative of the formula 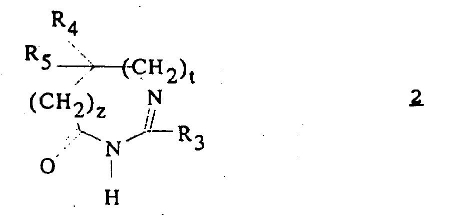 in which z, t, R₃, R₄ and R₅ are defined as indicated for (I) in claim 1, is reacted with a (biphenyl-4-yl)-methyl derivative 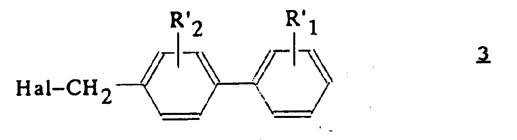 in which Hal is a halogen atom and R'₁ and R'₂ are respectively either R₁ and R₂ or a precursor of R₁ and/or R₂; and
b) the resulting compound of the formula 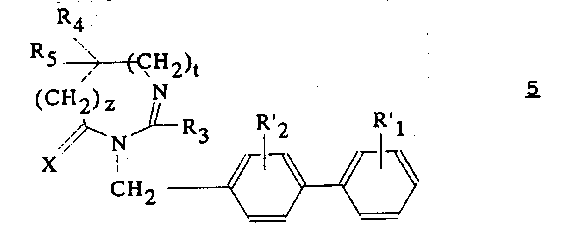 in which X is an oxygen atom, is treated so as to prepare the compound (I) by conversion of the groups R'₁ and/or R'₂ to R₁ and/or R₂ respectively.

5. A pharmaceutical composition in which a compound according to any one of Claims 1 to 3 is present as the active principle.

6. A pharmaceutical composition in which a compound according to any one of Claims 1 to 3 is present in association with a beta-blocking compound.

7. A pharmaceutical composition in which a compound according to any one of Claims 1 to 3 is present in association with a diuretic.

8. A pharmaceutical composition in which a compound according to any one of Claims 1 to 3 is present in association with a non-steroidal antiinflammatory

9. A pharmaceutical composition in which a compound according to any one of Claims 1 to 3 is present in association with a calcium antagonist.

10. A pharmaceutical composition in which a compound according to any one of Claims 1 to 3 is present in association with a tranquillizer.

## Claims (Claims for the following Contracting State(s) : ES)

1. A method of obtaining compounds of the formula 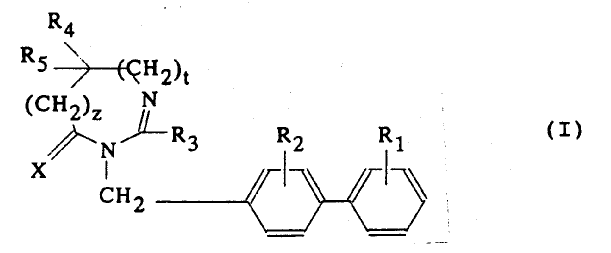 in which:
- R₁ is in the ortho position and is a nitrogen heterocycle selected from: 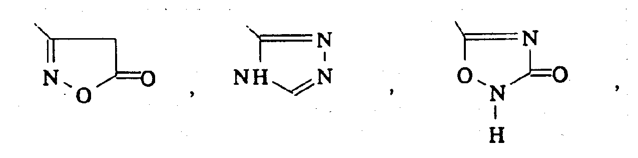 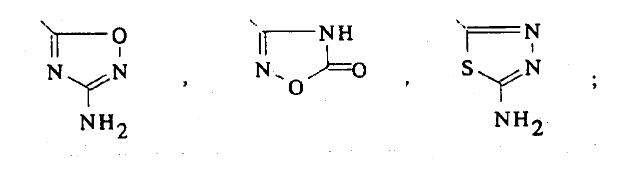
R₂ is hydrogen;
R₃ is a C₁-C₆ alkyl;
- R₄ and R₅, bonded together with the carbon to which they are attached, are a group of the formula (CH₂)ₙ;
- n is equal to 4 or 5;
- z and t are zero or z is 1 and t is zero; and
- X is an oxygen atom;
and their salts, characterized in that:
a) a heterocyclic derivative of the formula 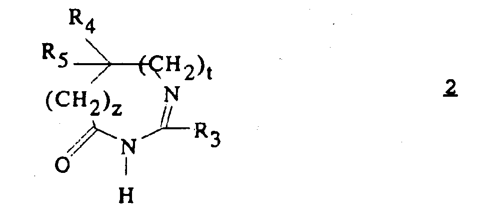 in which z, t, R₃, R₄ and R₅ are defined as indicated above, is reacted with a (biphenyl-4-yl)methyl derivative 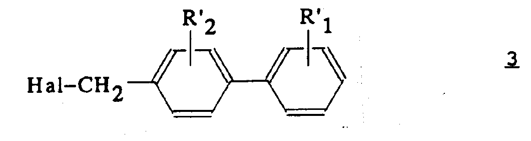 in which Hal is a halogen atom and R'₁ and R'₂ are respectively either R₁ and R₂ or a precursor of R₁ and/or R₂; and
b) the resulting compound of the formula 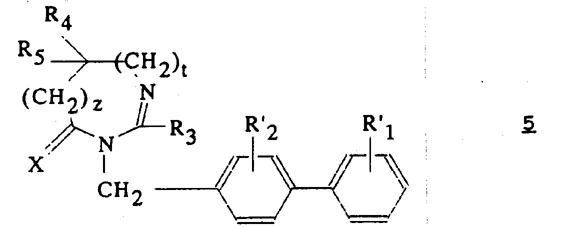 in which X is an oxygen atom, is treated so as to prepare the compound (I) by conversion of the groups R'₁ and/or R'₂ to R₁ and/or R₂ respectively.

2. A method according to Claim 1 for the preparation of a compound selected from:
- 2-n-butyl-1-[(2'-(5-oxo-1,2,4-oxadiazol-3-yl)biphenyl-4-yl)methyl]-4-spirocyclopentane-2-imidazolin-5-one;
1-[(2'-(4,5-dihydro-5-oxoisoxazol-3-yl)biphenyl-4-yl)methyl]-2-n-propyl-4-spirocyclohexane-2-imidazolin-5- one;
- 1-[(2'-(2-amino-1,3,4-thiadiazol-5-yl)biphenyl-4-yl)-methyl]-2-n-butyl-4-spirocyclopentane-2-imidazoline-5- one;
- 1-[(2'-(3-amino-1,2,4-oxadiazol-5-yl)biphenyl-4-yl)-methyl]-2-n-butyl-4-spirocyclopentane-2-imidazoline-5- one;
2-n-butyl-4-spirocyclopentane-1-[(2'-(1,2,4-triazol-3-yl)biphenyl-4-yl)methyl]-2-imidazolin-5-one; or
- 2-n-butyl-1-[(2'-(3-oxo-1,2,4-oxadiazol-5-yl)biphenyl-4-yl)methyl]-4-spirocyclopentane-2-imidazolin-5-one, characterized in that:
(i) a derivative 2 is used in which z = t = 0, R₃ is an n-propyl and CR₄R₅ is a cyclohexane or, if appropriate, R₃ is an n-butyl and CR₄R₅ is a cyclopentane; and
(ii) a derivative 3 is used in which R'₂ = H and
* R'₁ = -C(OC₂H₅)=NCO₂CH₃ if it is desired to obtain a compound (I) in which R₁ = 5-oxo-1,2,4-oxadiazol-3-yl;
* R'₁ = t-butoxycarbonyl if it is desired to obtain a compound (I) in which R₁ = 4,5-dihydro-5-oxoisoxazol-3-yl, 2-amino-1,3,4-thiadiazol-5-yl, 3-amino-1,2,4-oxadiazol-5-yl or 3-oxo-1,2,4-oxadiazol-5-yl; or
* R'₁ = trityl-1,2,4-triazol-5-yl if it is desired to obtain a compound (I) in which R1 = 1,2,4-triazol-5-yl.

3. A method of preparing a compound selected from:
- ethyl 4'-[(2-n-butyl-5-oxo-4-spirocyclopentane)-2-imidazolin-1-yl]methylbiphenyl-2-yl-(N-methoxycarbonyl)-carboximidate;
ethyl3-[(4'-(2-n-propyl-5-oxo-4-spirocyclohexane)-2-imidazolin-1-yl)methylbiphenyl-2-yl]-3-oxopropionate;
- 4'-[(2-n-butyl-5-oxo-4-spirocyclopentane)-2-imidazolin-1-yl]methylbiphenyl-2-carbonylthiosemicarbazide;
- 4'-[(2-n-butyl-5-oxo-4-spirocyclopentane)-2-imidazolin-1-yl]methylbiphenyl-2-carbonyloxyguanidine;
4'-[(2-n-butyl-5-oxo-4-spirocyclopentane)-2-imidazolin-1-yl]methylbiphenyl-2-carbonylsemicarbazide;
- (R,S)-2-n-butyl-4-cyclohexyl-4-methyl-1-[(2'-(5-oxo-1,2,4-oxadiazol-3-yl)biphenyl-4-yl)methyl]-2-imidazolin-5- one; or
- 2-n-butyl-4-cyclohexyl-4-methyl-1-[(2'-(5-oxo-1,2,4-oxadiazol-3-yl)biphenyl-4-yl)methyl]-2-imidazolin-5-one, dextrorotatory,
characterized in that:
a) a heterocyclic derivative of the formula 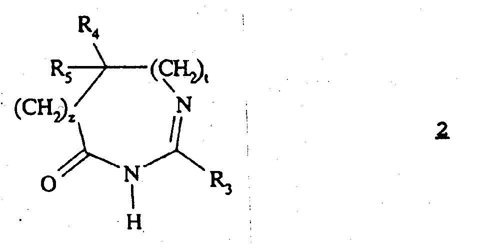 in which:
- z = t = 0; and
- R₃ is an n-propyl and CR₄R₅ is a cyclohexane or, if appropriate, R₃ is an n-butyl and CR₄R₅ is a cyclopentane, or else R₃ is an n-butyl, R₄ is a methyl and R₅ is a cyclohexyl,
is reacted with a (biphenyl-4-yl)methyl derivative 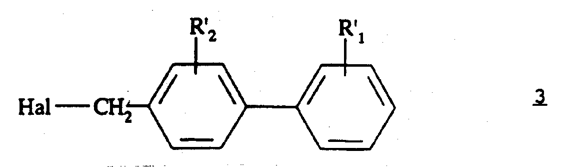 in which Hal is a halogen atom, R'₂ = H and
* R'₁ = -C(OC₂H₅)=NCO₂CH₃ if it is desired to obtain a compound (I) in which R1 is this group or a 5-oxo-1,2,4-oxadiazol-3-yl; or
^{*} R'₁ = t-butoxycarbonyl if it is desired to obtain a compound (I) in which R₁ = -COCH₂CO₂C₂H₅, - CONHNHCSNH₂, -COON=C(NH₂)₂ or -CONHNHCONH₂; and
b) the resulting compound of the formula 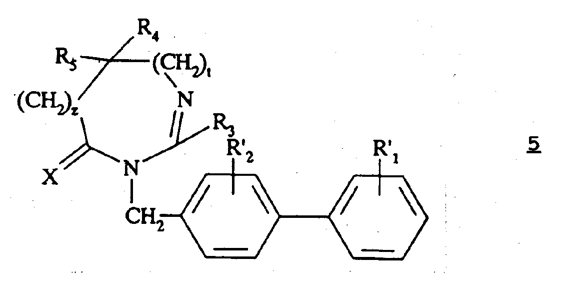 in which X is an oxygen atom, is treated so as to prepare the compound (I) by conversion of the groups R'₁ and/or R'₂ to R₁ and/or R₂ respectively.

4. A method of preparing pharmaceutical compositions in which a compound prepared by the method of any one of Claims 1 to 3 is present as the active principle, characterized in that said active principle is mixed with a pharmaceutically acceptable vehicle.

5. A method according to Claim 4 characterized in that the active principle is used in association with a beta-blocking compound.

6. A method according to Claim 4 characterized in that the active principle is used in association with a diuretic.

7. A method according to Claim 4 characterized in that the active principle is used in association with a non-steroidal antiinflammatory.

8. A method according to Claim 4 characterized in that the active principle is used in association with a calcium antagonist.

9. A method according to Claim 4 characterized in that the active principle is used in association with a tranquillizer.

## Claims (Claims for the following Contracting State(s) : GR)

1. A compound of the formula 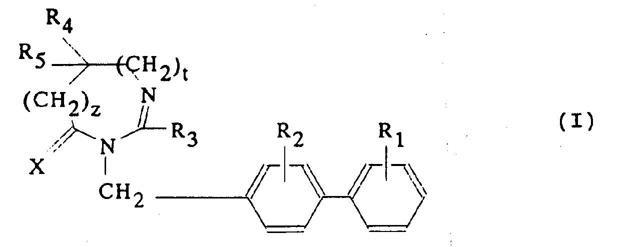 in which:
R₁ is in the ortho position and is a nitrogen heterocycle selected from: 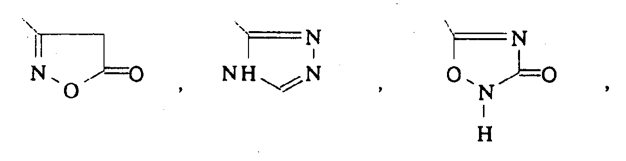 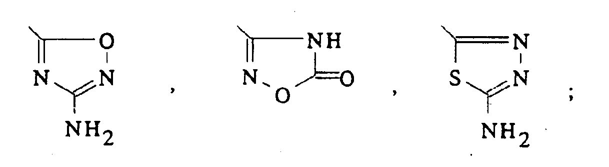
- R₂ is hydrogen; - R₂ is hydrogen;
- R₃ is a C₁-C₆ alkyl;
R₄ and R₅, bonded together with the carbon to which they are attached, are a group of the formula (CH₂)ₙ;
- n is equal to 4 or 5;
- z and t are zero or z is 1 and t is zero; and
X is an oxygen atom;
and its salts.

2. A compound according to Claim 1 which is selected from:
- 2-n-butyl-1-[(2'-(5-oxo-1,2,4-oxadiazol-3-yl)biphenyl-4-yl)methyl]-4-spirocyclopentane-2-imidazolin-5-one;
- 1-[(2'-(4,5-dihydro-5-oxoisoxazol-3-yl)biphenyl-4-yl)methyl]-2-n-propyl-4-spirocyclohexane-2-imidazolin-5- one;
- 1-[(2'-(2-amino-1,3,4-thiadiazol-5-yl)biphenyl-4-yl)-methyl]-2-n-butyl-4-spirocyclopentane-2-imidazolin-5-one;
- 1-[(2'-(3-amino-1,2,4-oxadiazol-5-yl)biphenyl-4-yl)-methyl]-2-n-butyl-4-spirocyclopentane-2-imidazolin-5-one;
- 2-n-butyl-4-spirocyclopentane-1-[(2'-(1,2,4-triazol-3-yl)biphenyl-4-yl)methyl]-2-imidazolin-5-one; or 2-n-butyl-1-[(2'-(3-oxo-1,2,4-oxadiazol-5-yl)biphenyl-4-yl)methyl]-4-spirocyclopentane-2-imidazolin-5-one.

3. A compound selected from:
- ethyl 4'-[(2-n-butyl-5-oxo-4-spirocyclopentane)-2-imidazolin-1-yl]methylbiphenyl-2-yl-(N-methoxycarbonyl)carboximidate;
ethyl3-[(4'-(2-n-propyl-5-oxo-4-spirocyclohexane)-2-imidazolin-1-yl)methylbiphenyl-2-yl]-3-oxopropionate;
- 4'-[(2-n-butyl-5-oxo-4-spirocyclopentane)-2-imidazolin-1-yl]methylbiphenyl-2-carbonylthiosemicarbazide;
- 4'-[(2-n-butyl-5-oxo-4-spirocyclopentane)-2-imidazolin-1-yl]methylbiphenyl-2-carbonyloxyguanidine;
4'-[(2-n-butyl-5-oxo-4-spirocyclopentane)-2-imidazolin-1-yl]methylbiphenyl-2-carbonylsemicarbazide;
- (R,S)-2-n-butyl-4-cyclohexyl-4-methyl-1-[(2'-(5-oxo-1,2,4-oxadiazol-3-yl)biphenyl-4-yl)methyl]-2-imidazolin-5- one; or
- 2-n-butyl-4-cyclohexyl-4-methyl-1-[(2'-(5-oxo-1,2,4-oxadiazol-3-yl)biphenyl-4-yl)methyl]-2-imidazolin-5- one, dextrorotatory

4. A method of preparing a compound (I) according to Claim 1, characterized in that:
a) a heterocyclic derivative of the formula 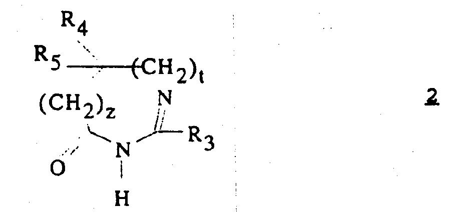 in which z, t, R₃, R₄ and R₅ are defined as indicated for (I) in Claim 1, is reacted with a (biphenyl-4-yl)-methyl derivative 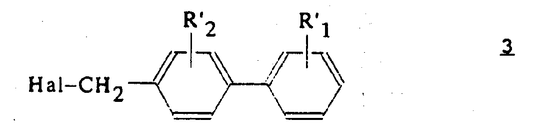 in which Hal is a halogen atom and R'₁ and R'₂ are respectively either R₁ and R₂ or a precursor of R₁ and/or R2;and
b) the resulting compound of the formula 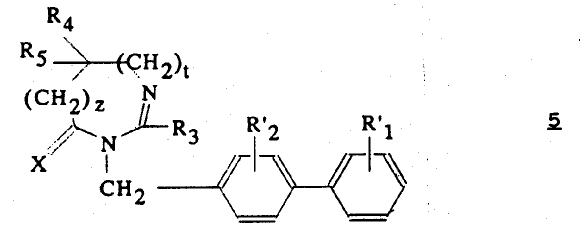 in which X is an oxygen atom, is treated so as to prepare the compound (I) by conversion of the groups R'₁ and/or R'₂ to R₁ and/or R₂ respectively.

5. A method of preparing a pharmaceutical composition in which a compound according to any one of Claims 1 to 3 is present as the active principle, characterized in that said active principle is mixed with a pharmaceutically acceptable vehicle.

6. A method according to Claim 5 characterized in that the active principle is used in association with a beta-blocking compound.

7. A method according to Claim 5 characterized in that the active principle is used in association with a diuretic.

8. A method according to Claim 5 characterized in that the active principle is used in association with a non-steroidal antiinflammatory.

9. A method according to Claim 5 characterized in that the active principle is used in association with a calcium antagonist.

10. A method according to Claim 5 characterized in that the active principle is used in association with a tranquillizer.

## Patentansprüche (Patentansprüche für folgende(n) Vertragsstaat(en) : AT, BE, CH, DE, DK, FR, GB, IT, LI, LU, NL, PT, SE)

1. Verbindung der Formel: 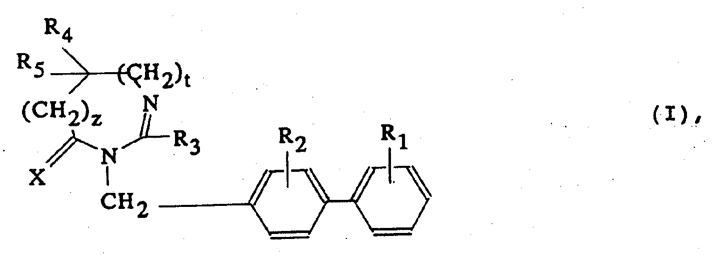 worin R₁ in ortho-Stellung vorliegt und einen Stickstoff-Heterocyclus, ausgewählt aus: 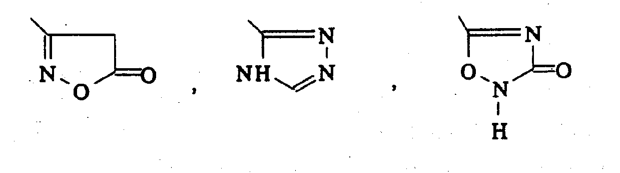 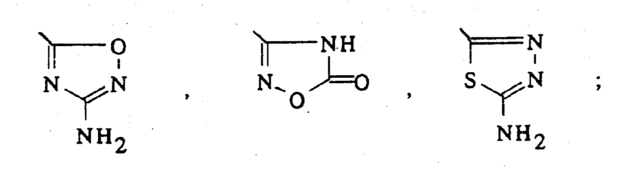 bedeutet; R₂ Wasserstoff darstellt; R₃ C₁-C₆-Alkyl ist; R₄ und R₅, gemeinsam mit dem Kohlenstoff, an den sie gebunden sind, verbunden, eine Gruppe der Formel (CH₂)ₙ bedeuten; n 4 oder 5 ist; z und t Null sind, oder z 1 ist, und t Null ist; X ein Sauerstoffatom darstellt; und ihre Salze.

2. Verbindung nach Anspruch 1, ausgewählt aus:
2-n-Butyl-1-[(2'-(5-oxo-1,2,4-oxadiazol-3-yl)-biphenyl-4-yl)-methyl]-4-spirocyclopentan-2-imidazolin-5-on;
1-[(2'-(4,5-Dihydro-5-oxoisoxazol-3-yl)-biphenyl-4-yl)-methyl]-2-n-propyl-4-spirocyclohexan-2-imidazolin-5- on;
1-[(2'-(2-Ami no-1,3,4-thiadiazol-5-yl)-biphenyl-4-yl)-methyl]-2-n-butyl-4-spi rocyclopentan-2-imidazolin-5-on;
1-[(2'-(3-Ami no-1,2,4-oxadiazol-5-yl)-biphenyl-4-yl)methyl]-2-n-butyl-4-spirocyclopentan-2-imidazolin-5-on;
2-n-Butyl-4-spirocyclopentan-1-[(2'-(1,2,4-triazol-3-yl)-biphenyl-4-yl)-methyl]-2-imidazolln-5-on; oder
2-n-Butyl-1-[(2'-(3-oxo-1,2,4-oxadiazol-5-yl)-biphenyl-4-yl)-methyl]-4-spirocyclopentan-2-imidazolln-5-on.

3. Verbindung, ausgewählt aus:
Ethyl-4'-[(2-n-butyl-5-oxo-4-spirocyclopentan)-2-imidazolin -1-yl]-methylbiphenyl-2-yl-(N-methoxycarbonyl)-carboximidat;
Ethyl-3-[(4'-(2-n-propyl-5-oxo-4-spirocyclohexan)-2-imidazolin-1-yl]-methylbiphenyl-2-yl]-3-oxopropionot;
4'-[(2-n-butyl-5-oxo-4-spirocyclopentan)-2-imidazolin-1-yl]-methylbiphenyl-2-carbonylthiosemicarbazid;
4'-[(2-n-butyl-5-oxo-4-spirocyclopentan)-2-imidazolin-1-yl]-methylbiphenyl-2-carbonyloxyguanidin;
4'-[(2-n-butyl-5-oxo-4-spirocyclopentan)-2-imidazolin-1-yl]-methylbiphenyl-2-carbonylsemicarbazid;
(R,S)-2-n-Butyl-4-cyclohexyl-4-methyl-1-[(2'-(5-oxo-1,2,4-oxadiazol-3-yl)-biphenyl-4-yl)-methyl]-2-imidazolin-5-on;
2-n-Butyl-4-cyclohexyl-4-methyl-1-[(2'-(5-oxo-1,2,4-oxadiazol-3-yl)-biphenyl-4-yl)-methyl]-2-imidazolin-5- on, rechtsdrehend.

4. Verfahren zur Herstellung einer Verbindung (I) nach Anspruch 1, dadurch gekennzeichnet, daß:
a) ein heterocyclisches Derivat der Formel: 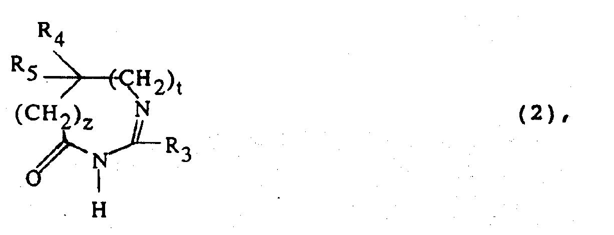 worin z, t, R₃, R₄ und R₅ die für (I) in Anspruch 1 angegebenen Bedeutungen haben, umgesetzt wird mit einem (Biphenyl-4-yl)-methyl-Derivat: 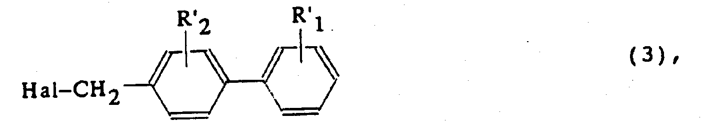 worin Hal ein Halogenatom bedeutet, und R'₁ und R'₂ jeweils entweder R₁ und R₂ oder einen Vorläufer von R₁ und/oder R₂ darstellen;
b) die so erhaltene Verbindung der Formel 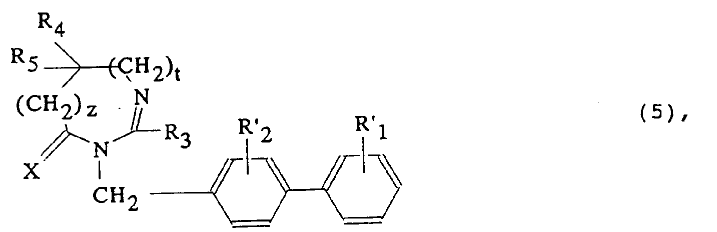 worin X ein Sauerstoffatom ist, behandelt wird, wobei die Verbindung (I) durch Transformation der Gruppen R'₁ und/oder R'₂ jeweils in R₁ und/oder R₂ hergestellt wird.

5. Pharmazeutische Zusammensetzung, welche als aktiven Bestandteil eine Verbindung nach einem der Ansprüche 1 bis 3 enthält.

6. Pharmazeutische Zusammensetzung, welche eine Verbindung nach einem der Ansprüche 1 bis 3 in Vereinigung mit einer β-Blocker-Verbindung enthält.

7. Pharmazeutische Zusammensetzung, welche eine Verbindung nach einem der Ansprüche 1 bis 3 in Vereinigung mit einem Diuretikum enthält.

8. Pharmazeutische Zusammensetzung, welche eine Verbindung nach einem der Ansprüche 1 bis 3 in Vereinigung mit einem Nicht-Steroid-Entzündungshemmer enthält.

9. Pharmazeutische Zusammensetzung, welche eine Verbindung nach einem der Ansprüche 1 bis 3 in Vereinigung mit einem Calcium-Antagonisten enthält.

10. Pharmazeutische Zusammensetzung, welche eine Verbindung nach einem der Ansprüche 1 bis 3 in Vereinigung mit einem Beruhigungsmittel enthält.

## Patentansprüche (Patentansprüche für folgende(n) Vertragsstaat(en) : ES)

1. Verfahren zum Erhalten von Verbindungen der Formel: 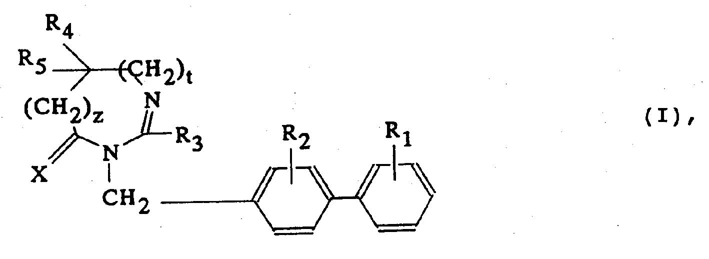 worin R₁ in ortho-Stellung vorliegt und einen Stickstoff-Heterocyclus, ausgewählt aus: 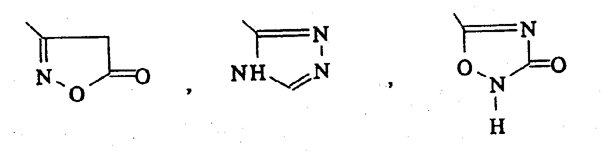 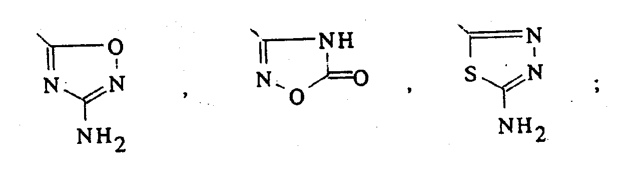 bedeutet; R₂ Wasserstoff darstellt; R₃ C₁-C₆-Alkyl ist; R₄ und R₅, gemeinsam mit dem Kohlenstoff, an den sie gebunden sind, verbunden, eine Gruppe der Formel (CH₂)ₙ bedeuten; n 4 oder 5 ist; z und t Null sind, oder z 1 ist, und t Null ist; X ein Sauerstoffatom darstellt; und ihrer Salze, dadurch gekennzeichnet, daß:
a) ein heterocyclisches Derivat der Formel: 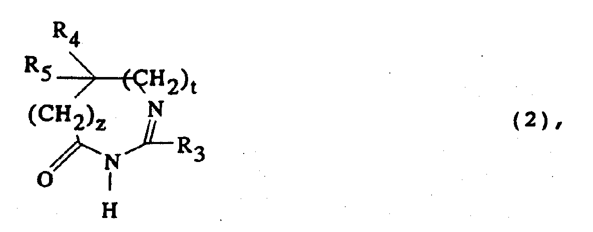 worin z, t, R₃, R₄ und R₅ die oben angegebenen Bedeutungen haben, umgesetzt wird mit einem (Biphenyi-4-yl)-methyl-Derivat: 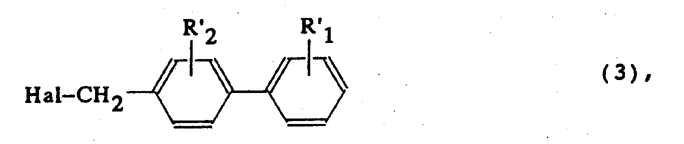 worin Hal ein Halogenatom bedeutet, und R'₁ und R'₂ jeweils entweder R₁ und R₂ oder einen Vorläufer von R₁ und/oder R₂ darstellen;
b) die so erhaltene Verbindung der Formel 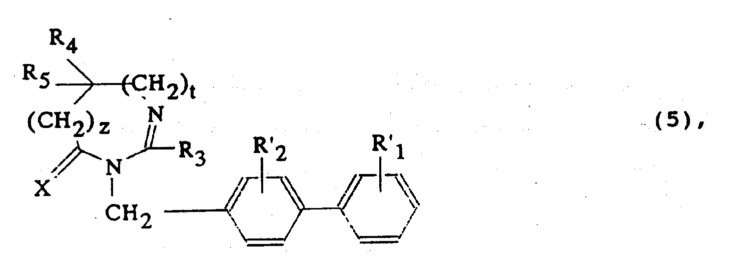 worin X ein Sauerstoffatom ist, behandelt wird, wobei die Verbindung (I) durch Transformation der Gruppen R'₁ und/oder R'₂ jeweils in R₁ und/oder R₂ hergestellt wird.

2. Verfahren nach Anspruch 1 zur Herstellung einer Verbindung, ausgewählt aus:
2-n-Butyl-1-[(2'-(5-oxo-1,2,4-oxadiazol-3-yl)-biphenyl-4-yl)-methyl]-4-spirocyclopentan-2-imidazolin-5-on;
1-[(2'-(4,5-Dihydro-5-oxoisoxazol-3-yl)-biphenyl-4-yl)-methyl]-2-n-propyl-4-spirocyclohexan-2-imidazolin-5- on;
1-[(2'-(2-Amino-1,3,4-thiadiazol-5-yl)-biphenyl-4-yl)-methyl]-2-n-butyl-4-spirocyclopentan-2-imidazolin-5-on;
1-[(2'-(3-Amino-1,2,4-oxadiazol-5-yl)-biphenyl-4-yl)methyl]-2-n-butyl-4-spirocyclopentan-2-imidazolin-5-on;
2-n-Butyl-4-spirocyclopentan-1-[(2'-(1,2,4-triazol-3-yl)-biphenyl-4-yl)-methyl]-2-imidazolin-5-on;oder
2-n-Butyl-1-[(2'-(3-oxo-1,2,4-oxadiazol-5-yl)-biphenyl-4-yl)-methyl]-4-spirocyclopentan-2-imidazolin-5-on, dadurch gekennzeichnet, daß verwendet werden:
(i) ein Derivat (2), worin z = t = Null; und R₃ n-Propyl darstellt, und CR₄R₅ Cyclohexan ist, oder gegebenenfalls R₃ n-Butyl bedeutet, und CR₄R₅ Cyclopentan darstellt; und
(ii) ein Derivat (3), worin R'₂ = H, und
^{*} R'₁ = -C(OC₂H₅) = NC0₂CH₃, wenn gewünscht wird, eine Verbindung (I) zu erhalten, worin R₁ = 5-Oxo-1,2,4-oxadiazol-3-yl;
^{*} R'₁ = tert.Butoxycarbonyl, wenn gewünscht wird, eine Verbindung (I) zu erhalten, worin R₁ = 4,5-Dihydro-5-oxoisoxazol-3-yl; 2-Amino-1,3,4-thiadiazol-5-yl; 3-Amino-1,2,4-oxadiazol-5-yl oder 3-0xo-1,2,4-oxadiazol-5-yl;
^{*} R'₁ = Trityl-1,2,4-triazol-5-yl, wenn gewünscht wird, eine Verbindung (I) zu erhalten, worin R₁ = 1,2,4-Triazol-5-yl.

3. Verfahren zur Herstellung einer Verbindung, ausgewählt aus:
Ethyl-4'-[(2-n-butyl-5-oxo-4-spirocyclopentan)-2-imidazolin -1-yl]-methylbiphenyl-2-yl-(N-methoxycarbonyl)-carboximidat;
Ethyl-3-[(4'-(2-n-propyl-5-oxo-4-spirocyclohexan)-2-imidazolin-1-yl]-methylbiphenyl-2-yl]-3-oxopropionat;
4'-[(2-n-butyl-5-oxo-4-spirocyclopentan)-2-imidazolin-1-yl]-methylbiphenyl-2-carbonylthiosemicarbazid;
4'-[(2-n-butyl-5-oxo-4-spirocyclopentan)-2-imidazolin-1-yl]-methylbiphenyl-2-carbonyloxyguanidin;
4'-[(2-n-butyl-5-oxo-4-spirocyclopentan)-2-imidazolin-1-yl]-methylbiphenyl-2-carbonylsemicarbazid;
(R,S)-2-n-Butyl-4-cyclohexyl-4-methyl-1-[(2'-(5-oxo-1,2,4-oxadiazol-3-yl)-biphenyl-4-yl)-methyl]-2-imidazolin-5-on; oder
2-n-Butyl-4-cyclohexyl-4-methyl-1-[(2'-(5-oxo-1,2,4-oxadiazol-3-yl)-biphenyl-4-yl)-methyl]-2-imidazolin-5- on, rechtsdrehend,
dadurch gekennzeichnet, daß:
a) ein heterocyclisches Derivat der Formel: 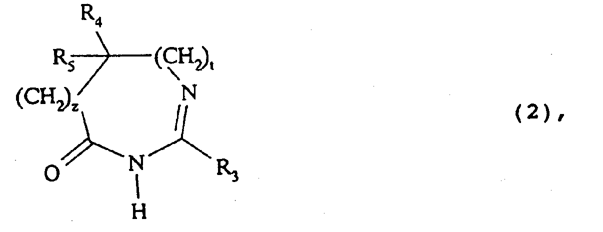 worin z = t = Null, R₃ n-Propyl bedeutet, und CR₄R₅ Cyclohexan darstellt, oder gegebenenfalls R₃ n-Butyl bedeutet, und CR₄R₅ Cyclopentan darstellt, oder auch R₃ n-Butyl bedeutet, R₄ Methyl darstellt, und R₅ Cyclohexyl ist; umgesetzt wird mit einem (Biphenyl-4-yl)-methyl-Derivat: 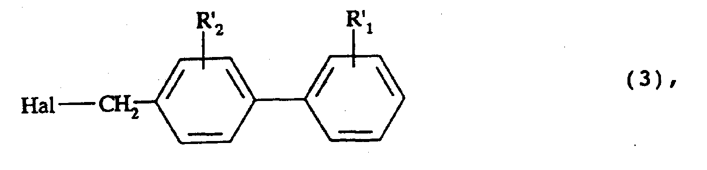 worin Hal ein Halogenatom bedeutet, R'₂ = H, und
^{*} R'₁ = -C(OC₂H₅) = NC0₂CH₃, wenn gewünscht wird, eine Verbindung (I) zu erhalten, worin R₁ diese Gruppe oder 5-Oxo-1,2,4-oxadiazol-3-yl bedeutet;
^{*} R'₁ = tert.Butoxycarbonyl, wenn gewünscht wird, eine Verbindung (I) zu erhalten, worin R₁ = COCH₂CO₂C₂H₅; -CONHNHCSNH₂; -COON = C(NH₂)₂ oder -CONHNHCONH₂;
b) die so erhaltene Verbindung der Formel 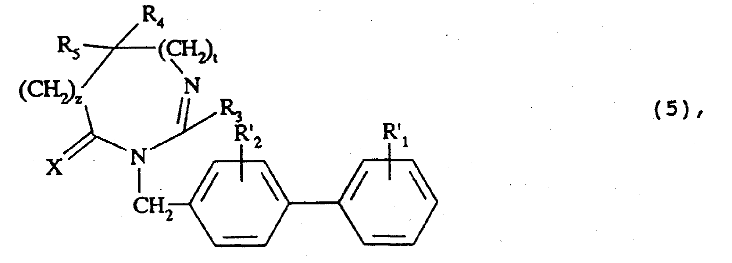 worin X ein Sauerstoffatom ist, behandelt wird, wobei die Verbindung (I) durch Transformation der Gruppen R'₁ und/oder R'₂ jeweils in R₁ und/oder R₂ hergestellt wird.

4. Verfahren zur Herstellung pharmazeutischer Zusammensetzungen, die als aktiven Bestandteil eine Verbindung enthalten, die gemäß dem Verfahren nach einem der Ansprüche 1 bis 3 hergestellt wurde, dadurch gekennzeichnet, daß der aktive Bestandteil mit einem pharmazeutisch annehmbaren Träger gemischt wird.

5. Verfahren nach Anspruch 4, dadurch gekennzeichnet, daß der aktive Bestandteil in Vereinigung mit einer P-Blokker-Verbindung verwendet wird.

6. Verfahren nach Anspruch 4, dadurch gekennzeichnet, daß der aktive Bestandteil in Vereinigung mit einem Diuretikum verwendet wird.

7. Verfahren nach Anspruch 4, dadurch gekennzeichnet, daß der aktive Bestandteil in Vereinigung mit einem Nicht-Steroid-Entzündungshemmer verwendet wird.

8. Verfahren nach Anspruch 4, dadurch gekennzeichnet, daß der aktive Bestandteil in Vereinigung mit einem Calcium-Antagonisten verwendet wird.

9. Verfahren nach Anspruch 4, dadurch gekennzeichnet, daß der aktive Bestandteil in Vereinigung mit einem Beruhigungsmittel verwendet wird.

## Patentansprüche (Patentansprüche für folgende(n) Vertragsstaat(en) : GR)

1. Verbindung der Formel: 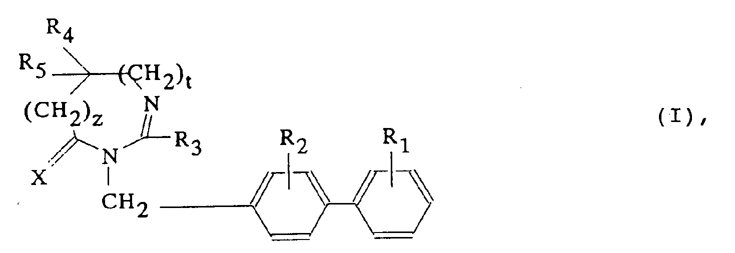 worin R₁ in ortho-Stellung vorliegt und einen Stickstoff-Heterocyclus, ausgewählt aus: 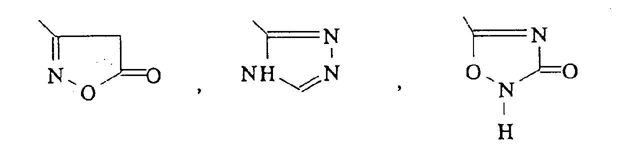 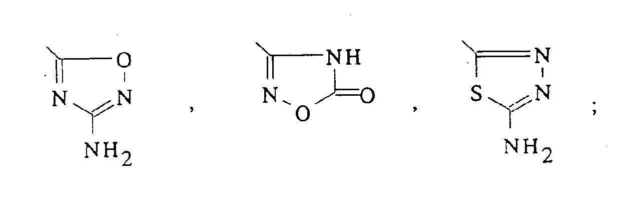 bedeutet; R₂ Wasserstoff darstellt; R₃ C₁-C₆-Alkyl ist; R₄ und R₅, gemeinsam mit dem Kohlenstoff, an den sie gebunden sind, verbunden, eine Gruppe der Formel (CH₂)ₙ bedeuten; n 4 oder 5 ist; z und t Null sind, oder z 1 ist, und t Null ist; X ein Sauerstoffatom darstellt; und ihre Salze.

2. Verbindung nach Anspruch 1, ausgewählt aus:
2-n-Butyl-1-[(2'-(5-oxo-1,2,4-oxadiazol-3-yl)-biphenyl-4-yl)-methyl]-4-spirocyclopentan-2-imidazol in-5-on;
1-[(2'-(4,5-Dihydro-5-oxoisoxazol-3-yl)-biphenyl-4-yl)-methyl]-2-n-propyl-4-spirocyclohexan-2-imidazolin-5- on;
1-[(2'-(2-Amino-1,3,4-thiadiazol-5-yl)-biphenyl-4-yl)-methyl]-2-n-butyl-4-spirocyclopentan-2-imidazolin-5-on;
1-[(2'-(3-Ami no-1,2,4-oxadiazol-5-yl)-biphenyl-4-yl)methyl]-2-n-butyl-4-spirocyclopentan-2-imidazolin-5-on;
2-n-Butyl-4-spirocyclopentan-1-[(2'-(1,2,4-triazol-3-yl)-biphenyl-4-yl)-methyl]-2-imidazolin-5-on;oder
2-n-Butyl-1-[(2'-(3-oxo-1,2,4-oxadiazol-5-yl)-biphenyl-4-yl)-methyl]-4-spirocyclopentan-2-imidazolin-5-on.

3. Verbindung, ausgewählt aus:
Ethyl-4'-[(2-n-butyl-5-oxo-4-spirocyclopentan)-2-imidazolin -1-yl]-methylbiphenyl-2-yl-(N-methoxycarbonyl)-carboximidat;
Ethyl-3-[(4'-(2-n-propyl-5-oxo-4-spirocyclopentan)-2-imidazolin-1-yl]-methylbiphenyl-2-yl]-3-oxopropionat;
4'-[(2-n-butyl-5-oxo-4-spirocyclopentan)-2-imidazolin-1-yl]-methylbiphenyl-2-carbonylthiosemicarbazid;
4'-[(2-n-butyl-5-oxo-4-spirocyclopentan)-2-imidazolin-1-yl]-methylbiphenyl-2-carbonyloxyguanidin;
4'-[(2-n-butyl-5-oxo-4-spirocyclopentan)-2-imidazolin-1-yl]-methylbiphenyl-2-carbonylsemicarbazid;
(R,S)-2-n-Butyl-4-cyclohexyl-4-methyl-1-[(2'-(5-oxo-1,2,4-oxadiazol-3-yl)-biphenyl-4-yl)-methyl]-2-imidazolin-5-on; oder
2-n-Butyl-4-cyclohexyl-4-methyl-1-[(2'-(5-oxo-1,2,4-oxadiazol-3-yl)-biphenyl-4-yl)-methyl]-2-imidazolin-5- on, rechtsdrehend.

4. Verfahren zur Herstellung einer Verbindung (I) nach Anspruch 1, dadurch gekennzeichnet, daß:
a) ein heterocyclisches Derivat der Formel: 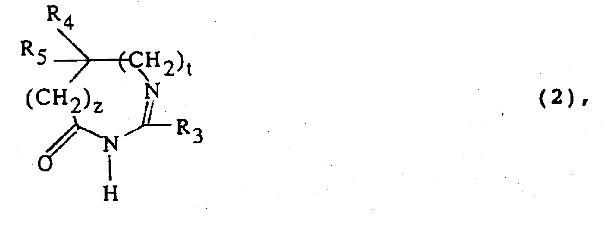 worin z, t, R₃, R₄ und R₅ die für (I) in Anspruch 1 angegebenen Bedeutungen haben, umgesetzt wird mit einem (Biphenyl-4-yl)-methyl-Derivat: 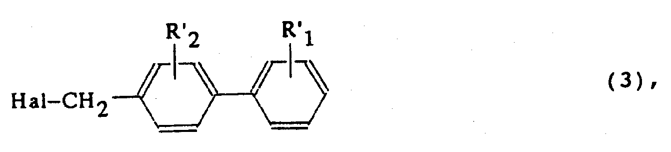 worin Hal ein Halogenatom bedeutet, und R'₁ und R'₂ jeweils entweder R₁ und R₂ oder einen Vorläufer von R₁ und/oder R₂ darstellen;
b) die so erhaltene Verbindung der Formel 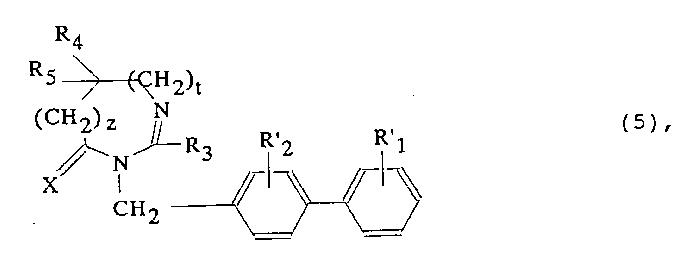 worin X ein Sauerstoffatom ist, behandelt wird, wobei die Verbindung (I) durch Transformation der Gruppen R'₁ und/oder R'₂ jeweils in R₁ und/oder R₂ hergestellt wird.

5. Verfahren zur Herstellung einer pharmazeutischen Zusammensetzung, die als aktiven Bestandteil eine Verbindung nach einem der Ansprüche 1 bis 3 enthält, dadurch gekennzeichnet, daß der aktive Bestandteil mit einem pharmazeutisch annehmbaren Träger gemischt wird.

6. Verfahren nach Anspruch 5, dadurch gekennzeichnet, daß der aktive Bestandteil in Vereinigung mit einer β-Blokker-Verbindung verwendet wird.

7. Verfahren nach Anspruch 5, dadurch gekennzeichnet, daß der aktive Bestandteil in Vereinigung mit einem Diuretikum verwendet wird.

8. Verfahren nach Anspruch 5, dadurch gekennzeichnet, daß der aktive Bestandteil in Vereinigung mit einem Nicht-Steroid-Entzündungshemmer verwendet wird.

9. Verfahren nach Anspruch 5, dadurch gekennzeichnet, daß der aktive Bestandteil in Vereinigung mit einem Calcium-Antagonisten verwendet wird.

10. Verfahren nach Anspruch 5, dadurch gekennzeichnet, daß der aktive Bestandteil in Vereinigung mit einem Beruhigungsmittel verwendet wird.
